# EUROPEAN PATENT APPLICATION

(11) **EP 1 959 021 A1**
(43) Date of publication of application: **20.08.2008**
(21) Application number: 06834313.6
(22) Date of filing: 08.12.2006
(51) Int. Cl.: C12Q 1/60, C12Q 1/26, C12Q 1/34, C12Q 1/44, G01N 33/92

(54) **METHOD FOR DETERMINATION OF CHOLESTEROL LEVEL IN REMNANT-LIKE LIPOPROTEIN, REAGENT AND KIT**

(30) Priority: 09.12.2005 JP 2005356586; 30.03.2006 JP 2006095152
(71) Applicant: KYOWA MEDEX CO., LTD., Chuo-ku, Tokyo 104-6004 (JP)
(72) Inventor: MISHIMA, Shingo, c/o Kyowa Medex Research Lab., KYOWA, Nagaizumi-cho, Sunto-gun, Shizuoka 411-0932 (JP); MIYAUCHI, Kazuhito, c/o Head Office, KYOWA, Tokyo 104-6004 (JP)
(74) Representative: Vossius & Partner
(86) International application number: PCT/JP2006/324558
(87) International publication number: WO 2007/066760

(57) **Abstract**

The present invention provides a method for simple and accurate measurement of cholesterol in remnant-like lipoprotein (RLP) (RLP-C) in a sample, which comprises the steps of: (i) eliminating cholesterol in lipoproteins other than RLP by reacting the sample with cholesterol oxidase, or cholesterol dehydrogenase and oxidized coenzyme, in an aqueous medium comprising surfactant A; (ii) reacting the reaction solution from which cholesterol in lipoproteins other than RLP has been eliminated in the above step (i) with cholesterol esterase and cholesterol oxidase, or cholesterol esterase, cholesterol dehydrogenase and oxidized coenzyme, in the presence of surfactant B to form hydrogen peroxide or reduced coenzyme; and (iii) measuring the hydrogen peroxide or reduced coenzyme formed in the above step (ii).

## Description

### Technical Field

The present invention relates to a method, a reagent and a kit for the measurement of cholesterol in remnant-like particles considered to be a risk factor for arteriosclerosis and the like in clinical tests.

### Background Art

Blood contains various kinds of lipoproteins. These lipoproteins are classified into chylomicron (hereinafter abbreviated as CM), very low-density lipoprotein (hereinafter abbreviated as VLDL), low-density lipoprotein (hereinafter abbreviated as LDL) and high-density lipoprotein (hereinafter abbreviated as HDL) according to their specific gravity. Also there exists intermediate-density lipoprotein (hereinafter abbreviated as IDL) whose density is between the densities of VLDL and LDL, which is a lipoprotein formed in the process of metabolism from VLDL to LDL. Each class of lipoprotein has its specific ratio of constituents such as cholesterol, triglycerides, phospholipids and proteins and has a different function in vivo.

In clinical tests, cholesterol in HDL is considered as a negative risk factor for arteriosclerosis and cholesterol in LDL is considered as a positive risk factor for arteriosclerosis. Thus, the measurement of cholesterol of such classes is frequently performed in the field of clinical testing.

In recent years, it has been demonstrated that cholesterol in lipoproteins formed by lipid metabolism and the like is a more closely linked risk factor for arteriosclerosis than LDL cholesterol. An example of the lipoprotein formed by lipid metabolism and the like is remnant-like particles (hereinafter abbreviated as RLP).

RLP is formed in the process of metabolism and decomposition of lipoproteins by the action of lipoprotein lipase, and is classified into chylomicron remnant (hereinafter abbreviated as CM remnant) and very low-density lipoprotein remnant (hereinafter abbreviated as VLDL remnant). IDL, that is included in VLDL remnant, is a lipoprotein having a specific gravity of 1.006 to 1.019 and is called VLDL remnant in a narrow sense.

RLP is a lipoprotein which is rich in triglycerides, and CM remnant has apoB-48 and apoE as major apoproteins and VLDL remnant has apoB-100 and apoE as major apoproteins.

A known example of a method for the measurement of cholesterol in RLP (hereinafter abbreviated as RLP-C) is a method in which RLP is separated from serum by immunochromatography using affinity gel containing anti-apoA-I monoclonal antibody and anti-apoB-100 monoclonal antibody, and cholesterol contained in the separated RLP is determined. A reagent for the determination of RLP-C applied in this method is commercially available from JIMRO Co., Ltd. (product: RLP-cholesterol "JIMRO" II) (see non-patent document Nos. 1 and 2). This method for the determination of RLP-C by immunoadsorption method is given health insurance scores by the Ministry of Health, Labour and Welfare in Japan. However, this method employs affinity chromatography using antibodies and requires separation of components of a sample, which makes it a cumbersome and time-consuming method.

Also known is a method for measuring RLP-C without separation of components of a sample, which comprises allowing surfactants, cholesterol oxidase or cholesterol dehydrogenase, cholesterol esterase and a phospholipid-hydrolyzing enzyme to act on a sample and measuring RLP-C in the sample (see patent document No. 1).
Patent document No. 1:
   Japanese Published Unexamined Patent Application No. 231597/01
Non-patent document No. 1:
   Arteriosclerosis, 25 (9, 10), 371-376 (1998)
Non-patent document No. 2:
   Clinical Chemistry, 48 (2), 217-219 (2002)

### Disclosure of the Invention

### Problems to be Solved by the Invention

An object of the present invention is to provide a method, a reagent and a kit for simple and accurate measurement of RLP-C in a sample.

### Means for Solving the Problems

The present invention relates to the following [1] to [27].
[1] A method for the measurement of cholesterol in remnant-like particles (hereinafter abbreviated as RLP) (hereinafter abbreviated as RLP-C) in a sample, which comprises carrying out the following steps (i) to (iii) successively:
   (i) eliminating cholesterol in lipoproteins other than RLP by reacting the sample with cholesterol oxidase, or cholesterol dehydrogenase and oxidized coenzyme, in an aqueous medium comprising the sample and a surfactant selected from the group consisting of polyoxyethylene alkyl ether phosphates, polyoxyethylene alkyl ether sulfuric acid ester salts, higher alcohol sulfuric acid ester salts, polyoxyethylene polycyclic phenyl ethers with an HLB value of less than 16.0, polyoxyethylene polycyclic phenyl ether sulfuric acid ester salts, polyoxyethylene-polyoxyalkylene polycyclic phenyl ethers, ethylenediamine-polyoxyethylene polyoxypropylene copolymers, polyoxyethylene alkylphenyl ethers, polyoxyethylene alkylphenyl ether sulfuric acid ester salts, polyoxyethylene short-chain branched alkyl ethers, polyoxyethylene-polyoxypropylene copolymers having a polyoxypropylene molecular weight of less than 2,750, polyoxyethylene-polyoxyalkylene short-chain branched alkyl ethers, polyoxyethylene-polyoxyalkylene alkyl ethers, bile acid derivatives, polyoxyethylene bisphenol-A-ethers, polyoxyethylene aryl ether sulfuric acid ester salts and polyoxypropylene meta-xylene diamines (hereinafter the surfactant is referred to as surfactant A);
   (ii) reacting the reaction solution from which cholesterol in lipoproteins other than RLP has been eliminated in the above step (i) with cholesterol esterase and cholesterol oxidase, or cholesterol esterase, cholesterol dehydrogenase and oxidized coenzyme, in the presence of a surfactant selected from the group consisting of polyoxyethylene-polyoxybutylene copolymers, polyoxyethylene polycyclic phenyl ethers with an HLB value of not less than 16.0, polyoxyethylene-polyoxyalkylene long-chain branched alkyl ethers, polyoxyethylene-polyoxypropylene copolymers having a polyoxypropylene molecular weight of not less than 2,750 and polyoxyethylene long-chain branched alkyl ethers (hereinafter the surfactant is referred to as surfactant B) to form hydrogen peroxide or reduced coenzyme; and
   (iii) measuring the hydrogen peroxide or reduced coenzyme formed in the above step (ii).
[2] The method according to [1], wherein step (i) is a step of eliminating cholesterol in lipoproteins other than RLP by reacting the sample with cholesterol dehydrogenase, oxidized coenzyme and reduced coenzyme oxidase in an aqueous medium comprising the sample and surfactant A.
[3] The method according to [1] or [2], wherein the reaction of step (i) is carried out in the presence of a polyanion.
[4] The method according to [3], wherein surfactant A is a surfactant selected from the group consisting of ethylenediamine-polyoxyethylene polyoxypropylene copolymers, polyoxyethylene alkylphenyl ethers, polyoxyethylene alkylphenyl ether sulfuric acid ester salts, polyoxyethylene short-chain branched alkyl ethers, polyoxyethylene-polyoxypropylene copolymers having a polyoxypropylene molecular weight of less than 2,750, polyoxyethylene-polyoxyalkylene short-chain branched alkyl ethers and bile acid derivatives [hereinafter the surfactant is referred to as surfactant (A1)].
[5] The method according to [1] or [2], wherein the reaction of step (i) is carried out in the presence of a polyanion and a divalent metal ion, or in the presence of an antibody selected from the group consisting of anti-apoB antibodies and anti-apoE antibodies.
[6] The method according to [5], wherein surfactant A is a surfactant selected from the group consisting of polyoxyethylene alkyl ether sulfuric acid ester salts, polyoxyethylene polycyclic phenyl ether sulfuric acid ester salts, polyoxyethylene-polyoxyalkylene polycyclic phenyl ethers, ethylenediamine-polyoxyethylene polyoxypropylene copolymers, polyoxyethylene alkylphenyl ethers, polyoxyethylene-polyoxypropylene copolymers having a polyoxypropylene molecular weight of less than 2,750, polyoxyethylene-polyoxyalkylene short-chain branched alkyl ethers, polyoxyethylene-polyoxyalkylene alkyl ethers and bile acid derivatives [hereinafter the surfactant is referred to as surfactant (A2)].
[7] The method according to any of [1] to [6], wherein step (i) is carried out in the presence of cholesterol esterase.
[8] The method according to any of [1] to [7], wherein step (ii) is carried out in the presence of a phospholipid-hydrolyzing enzyme.
[9] The method according to any of [1] to [8], wherein hydrogen peroxide is eliminated in the presence of a combination of peroxidase and one of the two oxidative coupling-type chromogens used for oxidative coupling coloring reaction, or in the presence of catalase.
[10] A reagent for the measurement of RLP-C in a sample comprising cholesterol esterase, cholesterol oxidase, a reagent for the elimination of hydrogen peroxide, a reagent for the measurement of hydrogen peroxide, surfactant A and surfactant B.
[11] A reagent for the measurement of RLP-C in a sample comprising cholesterol esterase, cholesterol dehydrogenase, oxidized coenzyme, surfactant A and surfactant B.
[12] A reagent for the measurement of RLP-C in a sample comprising cholesterol esterase, cholesterol dehydrogenase, oxidized coenzyme, reduced coenzyme oxidase, a reagent for the elimination of hydrogen peroxide, a reagent for the measurement of hydrogen peroxide, surfactant A and surfactant B.
[13] The reagent according to [10] or [12], wherein the reagent for the elimination of hydrogen peroxide is a reagent comprising peroxidase and one of the two oxidative coupling-type chromogens used for oxidative coupling coloring reaction, or a reagent comprising catalase.
[14] The reagent according to any of [10] to [13], further comprising a polyanion.
[15] The reagent according to [14], wherein surfactant A is surfactant (A1).
[16] The reagent according to any of [10] to [13], further comprising a reagent comprising a polyanion and a divalent metal ion, or a reagent comprising an antibody selected from the group consisting of anti-apoB antibodies and anti-apoE antibodies.
[17] The reagent according to [16], wherein surfactant A is surfactant (A2).
[18] The reagent according to any of [10] to [17], further comprising a phospholipid-hydrolyzing enzyme.
[19] A kit for the measurement of RLP-C in a sample which comprises a first reagent comprising cholesterol oxidase, a reagent for the elimination of hydrogen peroxide and surfactant A and a second reagent comprising a reagent for the measurement of hydrogen peroxide and surfactant B, said kit further comprising cholesterol esterase in either or both of the first reagent and the second reagent.
[20] A kit for the measurement of RLP-C in a sample which comprises a first reagent comprising cholesterol dehydrogenase, oxidized coenzyme and surfactant A and a second reagent comprising surfactant B, said kit further comprising cholesterol esterase in either or both of the first reagent and the second reagent.
[21] A kit for the measurement of RLP-C in a sample which comprises a first reagent comprising cholesterol dehydrogenase, oxidized coenzyme, reduced coenzyme oxidase, a reagent for the elimination of hydrogen peroxide and surfactant A and a second reagent comprising a reagent for the measurement of hydrogen peroxide and surfactant B, said kit further comprising cholesterol esterase in either or both of the first reagent and the second reagent.
[22] The kit according to [19] or [21], wherein the reagent for the elimination of hydrogen peroxide is a reagent comprising peroxidase and one of the two oxidative coupling-type chromogens used for oxidative coupling coloring reaction, or a reagent comprising catalase.
[23] The kit according to any of [19] to [22], wherein the first reagent further comprises a polyanion.
[24] The kit according to [23], wherein surfactant A is surfactant (A1).
[25] The kit according to any of [19] to [22], wherein the first reagent further comprises a polyanion and a divalent metal ion, or an antibody selected from the group consisting of anti-apoB antibodies and anti-apoE antibodies.
[26] The kit according to [25], wherein surfactant A is surfactant (A2).
[27] The kit according to any of [19] to [26], wherein the second reagent further comprises a phospholipid-hydrolyzing enzyme.

### Effect of the Invention

The present invention provides a method, a reagent and a kit for the simple and accurate measurement of RLP-C without separation of components of a sample.

### Brief Description of the Drawings

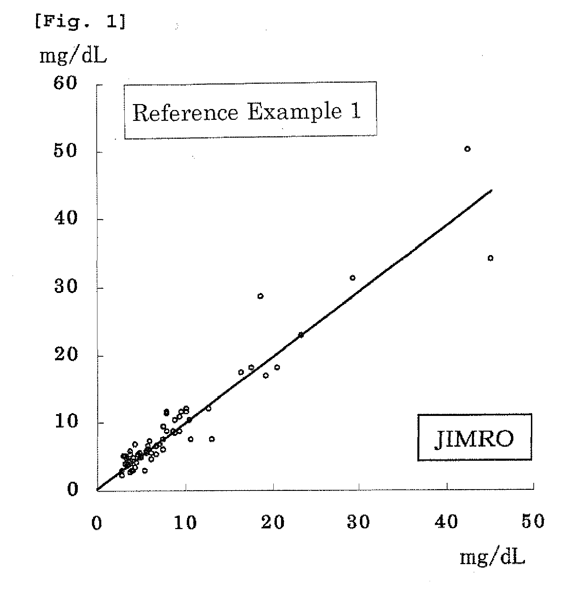

Fig. 1 is a graph showing the correlation between the measurement using the kit of Reference Example 1 and the measurement using RLP-cholesterol "JIMRO" II (JIMRO Co., Ltd.).

### Best Modes for Currying Out the Invention

In the present invention, the term "RLP" collectively refers to VLDL remnant and CM remnant. The term "RLP-C" collectively refers to free cholesterol and esterified cholesterol in RLP, that is, free cholesterol and esterified cholesterol in VLDL remnant, and free cholesterol and esterified cholesterol in CM remnant.

The method for the measurement of RLP-C of the present invention is characterized in that it does not require a step of separating components of a sample and it has a step of eliminating cholesterol in lipoproteins other than RLP prior to the measurement of RLP-C. Herein the term "lipoproteins other than RLP" in "eliminating cholesterol in lipoproteins other than RLP" refers to any of lipoproteins other than RLP, and also refers not only to all lipoproteins other than RLP, but also to one kind or plural kinds of lipoproteins other than RLP. Further, the term "cholesterol" in eliminating cholesterol in lipoproteins other than RLP" refers not only to a combination of all free cholesterol and all esterified cholesterol, but also to all or a part of free cholesterol, all or a part of esterified cholesterol, a combination of all free cholesterol and a part of esterified cholesterol, a combination of a part of free cholesterol and all esterified cholesterol, and a combination of a part of free cholesterol and a part of esterified cholesterol.

Examples of the samples used in the method of the present invention include whole blood, plasma, serum, spinal fluid, saliva, amniotic fluid, urine, sweat and pancreatic juice, among which plasma and serum are preferred.

There is no specific restriction as to the cholesterol esterase used in the present invention so long as it is an enzyme having the ability to hydrolyze cholesterol ester. For example, cholesterol esterase and lipoprotein lipase obtained from animals, plants or microorganisms, and those produced by genetic engineering techniques can be used.

As the cholesterol esterase, both unmodified ones and chemically modified ones can be used, and commercially available ones can also be used.

Examples of the commercially available cholesterol esterases include cholesterol esterase "Amano" 2 (CHE2; Amano Enzyme Inc.), cholesterol esterase "Amano" 3 (CHE3; Amano Enzyme Inc.), lipoprotein lipase "Amano" 6 (LPL6; Amano Enzyme Inc.), lipoprotein lipase (LPL-311; Toyobo Co., Ltd.), lipoprotein lipase (LPL-312; Toyobo Co., Ltd.), cholesterol esterase (COE-301; Toyobo Co., Ltd.), lipoprotein lipase (LPBP; Asahi Kasei Corporation), cholesterol esterase (CEBP-M; Asahi Kasei Corporation) and cholesterol esterase (CEN; Asahi Kasei Corporation). In the present invention, two or more kinds of cholesterol esterases can be used in combination.

Examples of the groups modifying cholesterol esterase (chemically modifying groups) used in chemical modification of cholesterol esterase include a group comprising polyethylene glycol as a main component, a group comprising polypropylene glycol as a main component, a group having a copolymer of polypropylene glycol and polyethylene glycol, a group comprising a water-soluble polysaccharide, a sulfopropyl group, a sulfobutyl group, a polyurethane group and a group having the chelating function, and preferred is a group comprising polyethylene glycol as a main component. Examples of the water-soluble polysaccharides include dextran, pullulan and soluble starch.

Examples of reagents for chemical modification of cholesterol esterase (chemical modifiers) include compounds that have both the above chemically modifying group and a functional group or a structure which can react with an amino group, a carboxyl group, a sulfhydryl group or the like of an enzyme. Examples of the functional groups or structures which can react with an amino group of an enzyme include a carboxyl group, an activated ester group (e.g., N-hydroxysuccinimide group), an acid anhydride, an acid chloride, an aldehyde, an epoxide group, 1,3-propanesultone and 1,4-butanesultone. An example of the functional group or structure which can react with a carboxyl group of an enzyme is an amino group. Examples of the groups or structures reactive with a sulfhydryl group of an enzyme include a maleimide group, a disulfide and α-haloester (e.g., α-iodo ester).

As the chemical modifiers, commercially available ones can be used. Examples of the commercially available chemical modifiers are Sunbright VFM-4101, Sunbright ME-050AS and Sunbright DE-030AS which have a group comprising polyethylene glycol as a main component and an N-hydroxysuccinimide group (all produced by NOF Corporation), Sunbright AKM series (e.g., Sunbright AKM-1510), Sunbright ADM series and Sunbright ACM series which have a group comprising polyalkylene glycol as a main component and an acid anhydride structure (all produced by NOF Corporation), EPOX-3400 and M-EPOX-5000 which have a group comprising polyethylene glycol as a main component and an epoxide group (both produced by Sheawater Polymers), and diethylenetriamine-N,N,N',N",N"-pentaacetic dianhydride which has a group having the chelating function and an acid anhydride structure (DTPA anhydride, Dojindo Laboratories).

Chemical modification of cholesterol esterase can be carried out, for example, by the following method, but is not limited thereto. First, cholesterol esterase is dissolved in a buffer of pH 8.0 or higher (e.g., HEPES buffer), and 0.01 to 500-fold molar amount of a chemical modifier is added thereto at 0 to 55°C, followed by stirring for 5 minutes to 5 hours. In the actual enzymatic reaction, this reaction mixture can be used as such, or if necessary, after removal of the unreacted chemical modifier with an ultrafilter, as the chemically modified cholesterol esterase.

There is no specific restriction as to the concentration of cholesterol esterase used in the method of the present invention, as long as the measurement of RLP-C according to the present invention can be performed. Its concentration in a reaction mixture is preferably 0.001 to 2000 U/mL, more preferably 0.005 to 1000 U/mL.

There is no specific restriction as to the cholesterol oxidase used in the present invention so long as it is an enzyme having the ability to oxidize cholesterol to form hydrogen peroxide. For example, cholesterol oxidase obtained from animals, plants or microorganisms, and those produced by genetic engineering techniques can be used. Commercially available ones such as cholesterol oxidase "Amano" 1 (CHOD1; Amano Enzyme Inc.), cholesterol oxidase (CHOPE; Kikkoman Corporation), cholesterol exidase (CHO-CE; Kikkoman Corporation) and cholesterol oxidase (COO321; Toyobo Co., Ltd.) can also be used. In the present invention, two or more kinds of cholesterol oxidases can be used in combination.

Cholesterol oxidase may be either an unmodified one or a chemically modified one. Chemically modified cholesterol oxidase can be prepared, for example, by the above method for chemical modification using the above chemical modifier.

There is no specific restriction as to the concentration of cholesterol oxidase used in the method of the present invention, as long as the measurement of RLP-C according to the present invention can be performed. Its concentration in a reaction mixture is preferably 0.001 to 2000 U/mL, more preferably 0.005 to 1000 U/mL.

There is no specific restriction as to the cholesterol dehydrogenase used in the present invention so long as it is an enzyme having the ability to oxidize cholesterol in the presence of oxidized coenzyme to form reduced coenzyme. For example, cholesterol dehydrogenase obtained from animals, plants or microorganisms, and those produced by genetic engineering techniques can be used. Commercially available ones such as cholesterol dehydrogenase "Amano" 5 (CHDH5; Amano Enzyme Inc.) can also be used. In the present invention, two or more kinds of cholesterol dehydrogenases can be used in combination. Cholesterol dehydrogenase may be either an unmodified one or a chemically modified one. Chemically modified cholesterol dehydrogenase can be prepared, for example, by the above method for chemical modification using the above chemical modifier.

There is no specific restriction as to the concentration of cholesterol dehydrogenase used in the method of the present invention, as long as the measurement of RLP-C according to the present invention can be performed. Its concentration in a reaction mixture is preferably 0.001 to 2000 U/mL, more preferably 0.005 to 1000 U/mL.

Examples of the oxidized coenzymes used in the measurement using cholesterol dehydrogenase are NAD(P)⁺ and thio-NAD(P)⁺. Examples of the reduced coenzymes formed by the reaction of cholesterol dehydrogenase are NAD(P)H and thio-NAD(P)H.

There is no specific restriction as to the concentration of oxidized coenzyme used in the method of the present invention, as long as the measurement of RLP-C according to the present invention can be performed. Its concentration in a reaction mixture is preferably 0.01 to 400 mmol/L, more preferably 0.1 to 100 mmol/L.

There is no specific restriction as to the reduced coenzyme oxidase used in the present invention so long as it is an enzyme having the ability to form hydrogen peroxide from reduced coenzyme formed by the reaction of cholesterol dehydrogenase. An example of the enzyme is NAD(P)H oxidase. As the reduced coenzyme oxidase, commercially available enzymes can also be used. An example of the commercially available reduced coenzyme oxidase is NADH Oxidase (Cosmo Bio Co., Ltd.)

There is no specific restriction as to the concentration of reduced coenzyme oxidase used in the method of the present invention, as long as the measurement of RLP-C according to the present invention can be performed. Its concentration in a reaction mixture is preferably 0.01 to 400 U/mL, more preferably 0.02 to 200 U/mL.

There is no specific restriction as to the phospholipid-hydrolyzing enzyme used in the present invention so long as it is an enzyme having the ability to hydrolyze phospholipids. For example, phospholipase D, phospholipase C, phospholipse A2 and lysophospholipase obtained from animals, plants or microorganisms can be used, and preferred are phospholipase D and phospholipase A2.

As the phospholipid-hydrolyzing enzyme, commercially available ones can also be used. Examples of the commercially available phospholipid-hydrolyzing enzymes are phospholipase D (PLD; Asahi Kasei Corporation), phospholipase D (PLDP: Asahi Kasei Corporation), phospholipase C (PLC; Asahi Kasei Corporation) and phospholipase A2 (PLA2; Asahi Kasei Corporation).

There is no specific restriction as to the concentration of phospholipid-hydrolyzing enzyme used in the method of the present invention, as long as the measurement of RLP-C according to the present invention can be performed. Its concentration in a reaction mixture is preferably 0.01 to 200 U/mL, more preferably 0.1 to 100 U/mL.

There is no specific restriction as to the polyanion used in the present invention as long as the measurement of RLP-C according to the present invention can be performed. Examples of the polyanion include dextran sulfate or its salt, heparin or its salt, phosphotungstic acid or its salt, sulfated cyclodextrin or its salt, sulfated oligosaccharide or its salt, and polycarboxylic acid-type polymers. Examples of the sulfated oligosaccharides are sulfated agarose, sulfated trehalose and chondroitin sulfate. Examples of the salts are sodium salt, potassium salt, lithium salt, ammonium salt and magnesium salt. In the present invention, two or more kinds of polyanions may be used. There is no specific restriction as to the concentration of polyanion used in the measurement of RLP-C of the present invention as long as the measurement of RLP-C according to the present invention can be performed. Its concentration in a reaction mixture is preferably 0.0001 to 10%, more preferably 0.001 to 1%.

There is no specific restriction as to the divalent metal ion used in the present invention as long as the measurement of RLP-C according to the present invention can be performed. Examples of the divalent metal ions are copper ion, iron ion, manganese ion and magnesium ion. In the present invention, two or more kinds of divalent metal ions may be used. There is no specific restriction as to the concentration of divalent metal ion used in the measurement of RLP-C of the present invention as long as the measurement of RLP-C according to the present invention can be performed. Its concentration in a reaction mixture is preferably 0.0001 to 10%, more preferably 0.001 to 1%.

There is no specific restriction as to the anti-apoB antibody used in the present invention as long as the measurement of RLP-C according to the present invention can be performed. Examples of the anti-apoB antibodies are those binding to lipoproteins containing apoB-100 (e.g., LDL and VLDL remnant) (anti-apoB-100 antibodies) and those binding to lipoproteins containing apoB-48 (e.g., CM remnant) (anti-apoB-48 antibodies). As the anti-apoB antibody, commercially available products can be used. Examples of the commercially available anti-apoB antibodies are Anti apolipoprotein B antibody and Anti Apolipoprotein B100 antibody (both produced by Cosmo Bio Co., Ltd.).

There is no specific restriction as to the anti-apoE antibody used in the present invention as long as the measurement of RLP-C according to the present invention can be performed. Examples of the anti-apoE antibodies are those binding to lipoproteins containing apoE (e.g., chylomicron remnant and VLDL remnant). As the anti-apoE antibody, commercially available products can be used. An example of the commercially available anti-apoE antibody is Anti apolipoprotein E antibody (Cosmo Bio Co., Ltd.).

Examples of surfactant A used in the present invention are polyoxyethylene alkyl ether phosphates (hereinafter abbreviated as POE alkyl ether phosphates), polyoxyethylene alkyl ether sulfuric acid ester salts (hereinafter abbreviated as POE alkyl ether sulfuric acid ester salts), higher alcohol sulfuric acid ester salts, polyoxyethylene polycyclic phenyl ethers with an HLB value of less than 16.0 (hereinafter abbreviated as POE polycyclic phenyl ethers with an HLB value of less than 16.0), polyoxyethylene polycyclic phenyl ether sulfuric acid ester salts (hereinafter abbreviated as POE polycyclic phenyl ether sulfuric acid ester salts), polyoxyethylene-polyoxyalkylene polycyclic phenyl ethers (hereinafter abbreviated as POE-POA polycyclic phenyl ethers), ethylenediamine-polyoxyethylene polyoxypropylene copolymers (hereinafter abbreviated as ethylenediamine-POE·POP copolymers), polyoxyethylene alkylphenyl ethers (hereinafter abbreviated as POE alkylphenyl ethers), polyoxyethylene alkylphenyl ether sulfuric acid ester salts (hereinafter abbreviated as POE alkylphenyl ether sulfuric acid ester salts), polyoxyethylene short-chain branched alkyl ethers (hereinafter abbreviated as POE short-chain branched alkyl ethers), polyoxyethylene-polyoxypropylene copolymers having a polyoxypropylene molecular weight of less than 2,750 (hereinafter abbreviated as POE·POP copolymers having a POP molecular weight of less than 2,750), polyoxyethylene-polyoxyalkylene short-chain branched alkyl ethers (hereinafter POE·POA short-chain branched alkyl ethers), polyoxyethylene-polyoxyalkylene alkyl ethers (hereinafter abbreviated as POE·POA alkyl ethers), bile acid derivatives, polyoxyethylene bisphenol-A-ethers (hereinafter abbreviated as POE bisphenol-A-ethers), polyoxyethylene aryl ether sulfuric acid ester salts (hereinafter abbreviated as POE aryl ether sulfuric acid ester salts) and polyoxypropylene meta-xylene diamines (POP meta-xylene diamines).

The alkyl in the POE alkyl ether phosphates includes alkyl groups having 1 to 30 carbon atoms, such as methyl, ethyl, propyl, butyl, pentyl, hexyl, heptyl, octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, icosyl, heneicosyl, docosyl (behenyl), tricosyl, tetracosyl, pentacosyl, hexacosyl, heptacosyl, octacosyl, nonacosyl and triacontyl. Specific examples (commercial products) of the POE alkyl ether phosphates include Plysurf A208B, Plysurf A210G, Plysurf A215C, Plysurf A217E and Plysurf A219B (all produced by Dai-ichi Kogyo Seiyaku Co., Ltd.).

The alkyl in the POE alkyl ether sulfuric acid ester salts includes alkyl groups having 1 to 30 carbon atoms, such as methyl, ethyl, propyl, butyl, pentyl, hexyl, heptyl, octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, icosyl, heneicosyl, docosyl (behenyl), tricosyl, tetracosyl, pentacosyl, hexacosyl, heptacosyl, octacosyl, nonacosyl and triacontyl. Examples of commercial products of the POE alkyl ether sulfuric acid ester salts include Newcol 1105-SN, Newcol 1305-SN and Newcol 1703-SFD (all produced by Nippon Nyukazai Co., Ltd.), and Persoft EF, Persoft EP, Persoft EL, Persoft EK, Persoft EFT and Persoft EDO (all produced by NOF Corporation). Examples of commercial products of the higher alcohol sulfuric acid ester salts include Monogen, Monogen 170T, Monogen LH, Monogen T-423, Monogen Y-100 and Monogen Y-500 (all produced by Dai-ichi Kogyo Seiyaku Co., Ltd.).

The polycyclic phenyl in the POE polycyclic phenyl ethers with an HLB value of less than 16.0 includes phenyl groups substituted with two or more groups (substituents) each having one aromatic ring therein, phenyl groups substituted with one or plural groups (substituents) each having two or more aromatic rings therein, etc. Examples of the groups having one aromatic ring therein are benzyl and 1-(phenyl)ethyl. An example of the group having two or more aromatic rings therein is naphthyl.

Examples of commercial products of the POE polycyclic phenyl ethers with an HLB value of less than 16.0 are Emulgen A-60, Emulgen A-90 and Emulgen B-66 (all produced by Kao Corporation), BLAUNON DSP-9, BLAUNON DSP-12.5, BLAUNON TSP-5, BLAUNON TSP-7.5 and BLAUNON TSP-16 (all produced by Aoki Oil Industrial Co., Ltd.), and Newcol 610, Newcol 707, Newcol 710, Newcol 2609 and Newcol 2614 (all produced by Nippon Nyukazai Co., Ltd.).

The polycyclic phenyl in the POE polycyclic phenyl ether sulfuric acid ester salts includes phenyl groups substituted with two or more groups (substituents) each having one aromatic ring therein, phenyl groups substituted with one or plural groups (substituents) each having two or more aromatic rings therein, etc. Examples of the groups having one aromatic ring therein are benzyl and 1-(phenyl)ethyl. An example of the group having two or more aromatic rings therein is naphthyl. Examples of commercial products of the POE polycyclic phenyl ether sulfuric acid ester salts are Newcol 707-SF, Newcol 707-SFC, Newcol 707-SN, Newcol 723-SF and Newcol 740-SF (all produced by Nippon Nyukazai Co., Ltd.).

The polycyclic phenyl in the POE·POA polycyclic phenyl ethers includes phenyl groups substituted with two or more groups (substituents) each having one aromatic ring therein, phenyl groups substituted with one or plural groups (substituents) each having two or more aromatic rings therein, etc. Examples of the groups having one aromatic ring therein are benzyl and 1-(phenyl)ethyl. An example of the group having two or more aromatic rings therein is naphthyl. The polyoxyalkylene in the POE·POA polycyclic phenyl ethers includes polyoxyalkylenes other than polyoxyethylene, such as polyoxypropylene and polyoxybutylene. Examples of commercial products of the POE·POA polycyclic phenyl ethers are Newkalgen CP-120 and Newkalgen GP-120 (both produced by Takemoto Oil & Fat Co., Ltd.), and Newcol 707-F, Newcol 710-F, Newcol 714-F, Newcol 2608-F, Newcol 2616-F and Newcol 2618-F (all produced by Nippon Nyukazai Co., Ltd.).

Examples of commercial products of ethylenediamine-POE·POP copolymers are Pluronic TR-701, Pluronic TR-702, Pluronic TR-704 and Pluronic TR-913R (all produced by Adeka Corporation), and Ethylenediamine PO40EO40, Ethylenediamine PO52EO60 and Unilube 32TY65BI (all produced by NOF Croporation).

The alkyl in the POE alkylphenyl ethers includes alkyl groups such as nonyl and octyl. Examples of commercial products of the POE alkylphenyl ethers are Triton N57, Triton N60, Triton X100, Triton X102, Triton X114 and Triton X35 (all produced by SIGMA), Nikkol NP-10, Nikkol NP-15, Nikkol NP-18TX, Nikkol NP-20, Nikkol NP-7.5, Nikkol OP-10 and Nikkol OP-30 (all produced by Nikko Chemicals Co., Ltd.), Emulgen 810, Emulgen 903, Emulgen 905, Emulgen 906, Emulgen 909, Emulgen 910, Emulgen 911, Emulgen 913, Emulgen 920, Emulgen 930, Emulgen 931 and Emulgen 950 (all produced by Kao Corporation), Nonion HS-204.5, Nonion HS-206, Nonion HS-208, Nonion HS-210, Nonion HS-215, Nonion HS-220, Nonion HS-240, Nonion NS-204.5, Nonion NS-206, Nonion NS-208.5, Nonion NS-210, Nonion NS-212, Nonion NS-215, Nonion NS-220, Nonion NS-230, Nonion NS-240 and Nonion NS-270 (all produced by NOF Corporation), Morinol NP-40, Morinol NP-60, Morinol NP-85, Morinol NP-100, Morinol NP-120, Morinol NP-160, Morinol NP-300, Morinol NP-400, Morinol NP-600 and Morinol NP-800 (all produced by Morin Chemical Industries Co., Ltd), and Octapol 50, Octapol 60, Octapol 80 and Octapol 100 (all produced by Sanyo Chemical Industries, Ltd.).

The alkyl in the POE alkylphenyl ether sulfuric acid ester salts includes alkyl groups having 1 to 30 carbon atoms, such as methyl, ethyl, propyl, butyl, pentyl, hexyl, heptyl, octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, icosyl, heneicosyl, docosyl (behenyl), tricosyl, tetracosyl, pentacosyl, hexacosyl, heptacosyl, octacosyl, nonacosyl and triacontyl. Examples of commercial products of the POE alkylphenyl ether sulfuric acid ester salts are Hitenol N-07, Hitenol N-08 and Hitenol N-17 (all produced by Dai-ichi Kogyo Seiyaku Co., Ltd.).

The short-chain branched alkyl in the POE short-chain branched alkyl ethers includes branched alkyl groups having 6 to 12 carbon atoms, such as isohexyl, isoheptyl, isooctyl, isononyl, isodecyl, isoundecyl and isododecyl. Examples of commercial products of the POE short-chain branched alkyl ethers are Noigen SD110, Noigen SD150, Noigen SD300 and Noigen SD400 (all produced by Dai-ichi Kogyo Seiyaku Co., Ltd.).

Examples of commercial products of the POE·POP copolymers having a POP molecular weight of less than 2,750 are Pluronic 25R-1, Pluronic 25R-2, Pluronic 17R-1, Pluronic 17R-2, Pluronic 17R-3, Pluronic L-33, Pluronic L-34, Pluronic L-62, Pluronic L-64, Pluronic P-65, Pluronic F-68, Pluronic P-84, Pluronic P-85 and Pluronic F-88 (all produced by Adeka Corporation), Epan 420, Epan 720, Epan U103, Epan U105 and Epan U108 (all produced by Dai-ichi Kogyo Seiyaku Co., Ltd.), Morinol PP-100, Morinol PP-300 and Morinol PP-400 (all produced by Morin Chemical Industries Co., Ltd.), and BLAUNON P-101, BLAUNON P-106, BLAUNON P-171, BLAUNON P-172, BLAUNON P-174, BLAUNON P-201, BLAUNON EP-0840, BLAUNON EP-0480, BLAUON EP-0670 and BLAUNON EP-1461 (all produced by Aoki Oil Industrial Co., Ltd.).

The polyoxyalkylene in the POE·POA short-chain branched alkyl ethers includes polyoxyalkylenes other than polyoxyethylene, such as polyoxypropylene and polyoxybutylene. The short-chain branched alkyl in the POE·POA short-chain branched alkyl ethers includes branched alkyl groups having 6 to 19 carbon atoms, such as isohexyl, isoheptyl, isooctyl, isononyl, isodecyl, isoundecyl, isododecyl, isotridecyl, isotetradecyl, isopentadecyl, isohexadecyl, hexyldecyl, isoheptadecyl, isooctadecyl and isononadecyl. Examples of commercial products of the POE·POA short-chain branched alkyl ethers are Finesurf IDEP-604, Finesurf IDEP-608, Finesurf IDEP-802, Wondersurf ID-50, Wondersurf ID-70 and Wondersurf ID-90 (all produced by Aoki Oil Industrial Co., Ltd.), and Noigen XL-140, Noigen XL-160, Noigen XL-400 and Noigen XL-1000F (all produced by Dai-ichi Kogyo Seiyaku Co., Ltd.).

The polyoxyalkylene in the POE·POA alkyl ethers includes polyoxyalkylenes other than polyoxyethylene, such as polyoxypropylene and polyoxybutylene. The alkyl in the POE·POA alkyl ethers includes alkyl groups having 1 to 30 carbon atoms, such as methyl, ethyl, propyl, butyl, pentyl, hexyl, heptyl, octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, icosyl, heneicosyl, docosyl (behenyl), tricosyl, tetracosyl, pentacosyl, hexacosyl, heptacosyl, octacosyl, nonacosyl and triacontyl. Examples of commercial products of the POE·POA alkyl ethers are EMALEX DAPE-207, EMALEX DAPE-210, EMALEX DAPE-212, EMALEX DAPE-215, EMALEX DAPE-220 and EMALEX DAPE-230 (all produced by Nihon Emulsion Co., Ltd.), Emulgen LS-110 and Emulgen MS-110 (both produced by Kao Corporation), Wondersurf RL-80, Wondersurf RL-100, Wondersurf RL-140, Wondersurf S-800, Wondersurf S-1000 and Wondersurf S-1400 (all produced by Aoki Oil Industrial Co., Ltd.), and Unisafe PKA-5015, Unisafe PKA-5016, Unisafe PKA-5017 and Unisafe LM-2 (all produced by NOF Corporation).

Examples of the bile acid derivatives include cholic acid or its salt, taurocholic acid or its salt, glycocholic acid or its salt, lithocholic acid or its salt, deoxycholic acid or its salt, chenodeoxycholic acid or its salt, ursodeoxycholic acid or its salt, 7-oxolithocholic acid or its salt, 12-oxolithocholic acid or its salt, 12-oxochenodeoxycholic acid or its salt, 7-oxodeoxycholic acid or its salt, hyocholic acid or its salt, hyodeoxycholic acid or its salt, dehydrocholic acid or its salt, CHAPS {3[(3-cholamidopropyl)]dimethylammonio]propanesulfonic acid; Dojindo Laboratories}, CHAPSO {3[(3-cholamidopropyl)]dimethylammonio]-2-hydroxypropanesulfonic acid; Dojindo Laboratories}, BIGCHAP [N,N-bis(3-D-gluconamidopropyl)cholamide; Dojindo Laboratories] and deoxy-BIGCHAP [N,N-bis(3-D-gluconamidopropyl)deoxycholamide; Dojindo Laboratories]. Examples of the salts are ammonium salt, lithium salt, sodium salt, potassium salt, magnesium salt and calcium salt.

Examples of commercial products of the POE bisphenol-A-ethers are BLAUNON BEO-6, BLAUNON BEO-10 and BLAUNON BEO-17.5 (all produced by Aoki Oil Industrial Co., Ltd.).

The aryl in the POE aryl ether sulfuric acid ester salts includes aryl groups such as phenyl and naphthyl. Examples of commercial products of the POE aryl ether sulfuric acid ester salts are Newcol B4-SN and Newcol B13-SN (both produced by Nippon Nyukazai Co., Ltd.).

Examples of commercial products of the POP meta-xylene diamines are BLAUNON MXDA-PO1, BLAUNON MXDA-PO2 and BLAUNON MXDA-PO4 (all produced by Aoki oil Industrial Co., Ltd.).

In the present invention, surfactant A, that is, a surfactant which is selected from the group consisting of POE alkyl ether phosphates, POE alkyl ether sulfuric acid ester salts, higher alcohol sulfuric acid ester salts, POE polycyclic phenyl ethers with an HLB value of less than 16.0, POE polycyclic phenyl ether sulfuric acid ester salts, POE·POA polycyclic phenyl ethers, ethylenediamine-POE·POP copolymers, POE alkylphenyl ethers, POE alkylphenyl ether sulfuric acid ester salts, POE short-chain branched alkyl ethers, POE·POP copolymers having a POP molecular weight of less than 2,750, POE·POA short-chain branched alkyl ethers, POE·POA alkyl ethers, bile acid derivatives, POE bisphenol-A-ethers, POE aryl ether sulfuric acid ester salts and POP meta-xylene diamines can be used alone, that is, in the absence of a polyanion, a combination of a polyanion and a divalent metal ion, an anti-apoB antibody and an anti-apoE antibody.

In the present invention, surfactant (A1) is a group of surfactants that are selected from the group of surfactant A and can be used in the presence of a polyanion and in the absence of a combination of a polyanion and a divalent metal ion, or an anti-apoB antibody and an anti-apoE antibody. Examples of surfactant (A1) are ethylenediamine-POE·POP copolymers, POE alkylphenyl ethers, POE alkylphenyl ether sulfuric acid ester salts, POE short-chain branched alkyl ethers, POE·POP copolymers having a POP molecular weight of less than 2,750, POE·POA short-chain branched alkyl ethers and bile acid derivatives.

In the present invention, surfactant (A2) is a group of surfactants that are selected from the group of surfactant A and can be used in the presence of a polyanion and a divalent metal ion, or an antibody selected from the group consisting of anti-apoB antibodies and anti-apoE antibodies, and includes a part of surfactant (A1). Examples of surfactant (A2) are POE alkyl ether sulfuric acid ester salts, POE polycyclic phenyl ether sulfuric acid ester salts, POE·POA polycyclic phenyl ethers, ethylenediamine-POE·POP copolymers, POE alkylphenyl ethers, POE·POP copolymers having a POP molecular weight of less than 2,750, POE·POA short-chain branched alkyl ethers, POE·POA alkyl ethers and bile acid derivatives.

Surfactant (A1), which is a group of surfactants selected from the group of surfactant A as described above, can be appropriately selected, for example, by using reagents prepared based on the reagent composition described in Example 2, that is, reagents prepared by adding a polyanion (e.g., dextran sodium sulfate) to the major constituents in the reagent composition described in Example 2 and further adding various surfactants in place of Emulgen B-66, and carrying out examination according to the method of Example 77 or 78 of the present invention.

Surfactant (A2), which is a group of surfactants selected from the group of surfactant A as described above, can be appropriately selected, for example, by using reagents prepared based on the reagent composition described in Example 2, that is, reagents prepared by adding a combination of a polyanion and a divalent metal ion (e.g., a combination of dextran sodium sulfate and magnesium nitrate) or an antibody selected from the group consisting of anti-apoB antibodies and anti-apoE antibodies to the major constitutents in the reagent composition described in Example 2 and further adding various surfactants in place of Emulgen B-66, and carrying out examination according to the method of Example 77 or 78 of the present invention.

In the present invention, as surfactant A, surfactant (A1) and surfactant (A2), one kind or combinations of two or more kinds of surfactants selected from the respective surfactant groups can be used.

There is no specific restriction as to the concentration of surfactant A used in the measurement of RLP-C of the present invention, as long as the measurement of RLP-C according to the present invention can be performed. Its concentration in a reaction mixture is preferably 0.0001 to 5%, more preferably 0.0005 to 2%.

Examples of surfactant B used in the present invention are polyoxyethylene-polyoxybutylene copolymers (hereinafter abbreviated as POE·POB copolymers), polyoxyethylene polycyclic phenyl ethers with an HLB value of not less than 16.0 (hereinafter abbreviated as POE polycyclic phenyl ethers with an HLB value of not less than 16.0), polyoxyethylene-polyoxyalkylene long-chain branched alkyl ethers (hereinafter abbreviated as POE·POA long-chain branched alkyl ethers), polyoxyethylene-polyoxypropylene copolymers having a polyoxypropylene molecular weight of less than 2,750 (hereinafter abbreviated as POE·POP copolymers having a POP molecular weight of not less than 2,750) and polyoxyethylene long-chain branched alkyl ethers (hereinafter abbreviated as POE long-chain branched alkyl ethers). Surfactant B is a surfactant which is added to a reaction mixture after the elimination of cholesterol in lipoproteins other than RLP and enables the reaction of cholesterol in RLP with enzymes.

The molecular weight of the polyoxybutylene moiety of the POE·POB copolymers is preferably 700 or more, more preferably 1000 or more. Examples of commercial products of the POE·POB copolymers are Pronon B-204 and Pronon B-208 (both produced by NOF Corporation).

The polycyclic phenyl in the POE polycyclic phenyl ethers with an HLB value of not less than 16.0 includes phenyl groups substituted with two or more groups (substituents) each having one aromatic ring therein, phenyl groups substituted with one or plural groups (substituents) each having two or more aromatic rings therein, etc. Examples of the groups having one aromatic ring therein are benzyl and 1-(phenyl)ethyl. An example of the group having two or more aromatic rings therein is naphthyl. Examples of commercial products of the POE polycyclic phenyl ethers with an HLB value of not less than 16.0 are BLAUNON TSP-50 (Aoki Oil Industrial Co., Ltd.), Emulgen A-500 (Kao Corporation) and Newcol 740 (Nippon Nyukazai Co., Ltd.).

The long-chain branched alkyl in the POE·POA long-chain branched alkyl ethers includes branched alkyl groups having 20 to 30 carbon atoms, such as isoicosyl, octyldodecyl, isoheneicosyl, isodocosyl, isotricosyl, isotetracosyl, decyltetradecyl, isopentacosyl, decylpentadecyl, isohexacosyl, dodecyltetradecyl, isoheptacosyl, isooctacosyl, isononacosyl and isotriacontyl. Examples of commercial products of the POE·POA long-chain branched alkyl ethers are Unilube MT-0620B, Unilube 20MT-2000B and Unilube 50MT-2200B (all produced by NOF Corporation).

Examples of commercial products of the POE·POP copolymers having a POP molecular weight of not less than 2,750 are Pluronic L-101, Pluronic P-103, Pluronic F-108, Pluronic L-121 and Pluronic L-122 (all produced by Adeka Corporation), and BLAUNON P-303 and BLAUNON P-304 (both produced by Aoki Oil Industrial Co., Ltd.).

Preferred examples of the long-chain branched alkyl in the POE long-chain branched alkyl ethers are branched alkyl groups having 13 to 30 carbon atoms. Particularly preferred examples of the POE long-chain branched alkyl ethers used in the present invention are those in which the long-chain branched alkyl has 21 to 30 carbon atoms or whose HLB value is not less than 15.0. Examples of the branched alkyl groups having 13 to 30 carbon atoms are isotridecyl, isotetradecyl, isopentadecyl, isohexadecyl, hexyldecyl, isoheptadecyl, isooctadecyl, isononadecyl, isoicosyl, octyldodecyl, isoheneicosyl, isodocosyl, isotricosyl, isotetracosyl, decyltetradecyl, isopentacosyl, decylpentadecyl, isohexacosyl, dodecyltetradecyl, isoheptacosyl, isooctacosyl, isononacosyl and isotriacontyl. Examples of commercial products of the POE long-chain branched alkyl ethers are EMALEX 1615, EMALEX 1620, EMALEX 1625, EMALEX 1815, EMALEX 1820, EMALEX 1825, EMALEX OD-16, EMALEX OD-25, EMALEX 2415, EMALEX 2420, EMALEX 2425, EMALEX 2515, EMALEX 2520 and EMALEX 2525 (all produced by Nihon Emulsion Co., Ltd.), and Noigen TDS-500F (Dai-ichi Kogyo Seiyaku Co., Ltd.).

There is no specific restriction as to the concentration of surfactant B used in the measurement of RLP-C of the present invention, as long as the measurement of RLP-C according to the present invention can be performed. Its concentration in a reaction mixture is preferably 0.001 to 20%, more preferably 0.01 to 10%.

The HLB value refers to hydrophile-lipophile balance value. The HLB value of a sufractant used in the present invention, for example, that of POE polycyclic phenyl ether can be calculated by the methods described in Surfactant Handbook (Tokiyuki Yoshida, et al., Kogyo Tosho Co., Ltd.) and New Surfactants (Hiroshi Horiguchi, Sankyo Publishing Co., Ltd.). It is also possible to refer to HLB values described in catalogues and pamphlets released from manufacturers of various kinds of surfactants.

There is no specific restriction as to the reagent for the elimination of hydrogen peroxide, as long as it is a reagent converting hydrogen peroxide formed from lipoprotein cholesterol other than RLP-C into a substance which does not affect reaction for measuring hydrogen peroxide formed from RLP-C, and examples of the reagents are a reagent comprising catalase and a reagent comprising peroxidase and one of the two oxidative coupling-type chromogens used for oxidative coupling coloring reaction. Specific examples are couplers and anilines or phenols in oxidative coupling-type chromogens described below. In the present specification, the term "the other oxidative coupling-type chromogen" is used in pair with the term "one of the two oxidative coupling-type chromogens used for oxidative coupling coloring reaction". For example, when the former refers to a coupler, the latter refers to a phenol or an aniline, and when the former refers to a phenol or an aniline, the latter refers to a coupler.

When a reagent comprising catalase is used as the reagent for the elimination of hydrogen peroxide, the concentration of catalase is preferably 0.001 to 5000 kU/L, more preferably 0.01 to 1000 kU/L.

When peroxidase and one of the two oxidative coupling-type chromogens used for oxidative coupling coloring reaction are used as the reagent for the elimination of hydrogen peroxide, the concentration of peroxidase is preferably 0.01 to 500 kU/L, more preferably 1 to 100 kU/L, and the concentration of one of the two oxidative coupling-type chromogens used for oxidative coupling coloring reaction is preferably 0.01 to 10 g/L.

The reagent for the measurement of hydrogen peroxide is a reagent for converting the formed hydrogen peroxide into a detectable substance. Examples of the detectable substances are a dye and luminescence, and preferred is a dye. When the detectable substance is a dye, a reagent for the measurement of hydrogen peroxide comprises an oxidative coloring type chromogen and a peroxidative substance such as peroxidase. Examples of the oxidative coloring type chromogen are oxidative coloring type chromogens described below. When the detectable substance is luminescence, a reagent for the measurement of hydrogen peroxide comprises a chemiluminescent substance. Examples of the chemiluminescent substances are luminol, isoluminol, lucigenin and acridinium ester.

When a reagent comprising an oxidative coloring type chromogen and a peroxidative substance such as peroxidase is used as the reagent for the measurement of hydrogen peroxide, hydrogen peroxide can be determined by subjecting hydrogen peroxide to reaction with an oxidative coloring type chromogen in the presence of a peroxidative substance to form a dye and determining the formed dye. When a reagent for the measurement of hydrogen peroxide comprising a chemiluminescent substance is used, hydrogen peroxide can be determined by subjecting hydrogen peroxide to reaction with the chemiluminescent substance to generate luminescence and determining the generated luminescence.

Examples of the oxidative coloring type chromogens are leuco-type chromogens and oxidative coupling-type chromogens.

A leuco-type chromogen is a substance that is converted into a dye by itself in the presence of hydrogen peroxide and a peroxidative substance such as peroxidase. Examples of the leuco-type chromogens are 10-N-carboxymethylcarbamoyl-3,7-bis(dimethylamino)-10H-phenothiazine (CCAP), 10-N-methylcarbamoyl-3,7-bis(dimethylamino)-10H-phenothiazine (MCDP), N-(carboxymethylaminocarbonyl)-4,4'-bis(dimethylamino)diphenylamine sodium salt (DA-64), 4,4'-bis(dimethylamino)diphenylamine and bis[3-bis(4-chlorophenyl)methyl-4-dimethylaminophenyl]amine (BCMA).

An oxidative coupling-type chromogen is a substance that forms a dye by oxidative coupling of two compounds in the presence of hydrogen peroxide and a peroxidative substance such as peroxidase.

Examples of the combinations of the two compounds include combinations of a coupler and an aniline and combinations of a coupler and a phenol. Examples of the couplers are 4-aminoantipyrine (4-AA) and 3-methyl-2-benzothiazolinone hydrazine. Examples of the anilines are N-(3-sulfopropyl)aniline, N-ethyl-N-(2-hydroxy-3-sulfopropyl)-3-methylaniline (TOOS), N-ethyl-N-(2-hydroxy-3-sulfopropyl)-3,5-dimethylaniline (MAOS), N-ethyl-N-(2-hydroxy-3-sulfopropyl)-3,5-dimethoxyaniline (DAOS), N-ethyl-N-(3-sulfopropyl)-3-methylaniline (TOPS), N-(2-hydroxy-3-sulfopropyl)-3,5-dimethoxyaniline (HDAOS), N,N-dimethyl-3-methylaniline, N,N-di(3-sulfopropyl)-3,5-dimethoxyaniline, N-ethyl-N-(3-sulfopropyl)-3-methoxyaniline, N-ethyl-N-(3-sulfopropyl)aniline, N-ethyl-N-(3-sulfopropyl)-3,5-dimethoxyaniline, N-(3-sulfopropyl)-3,5-dimethoxyaniline, N-ethyl-N-(3-sulfopropyl)-3,5-dimethylaniline, N-ethyl-N-(2-hydroxy-3-sulfopropyl)-3-methoxylaniline, N-ethyl-N-(2-hydroxy-3-sulfopropyl)aniline, N-ethyl-N-(3-methylphenyl)-N'-succinylethylenediamine (EMSE), N-ethyl-N-(3-methylphenyl)-N'-acetylethylenediamine and N-ethyl-N-(2-hydroxy-3-sulfopropyl)-4-fluoro-3,5-dimethoxyaniline (F-DAOS). Examples of the phenols are phenol, 4-chlorophenol, 3-methylphenol and 3-hydroxy-2,4,6-triiodobenzoic acid (HTIB).

In the measurement of hydrogen peroxide, the concentration of a peroxidative substance is not specifically limited as long as it is suited for the measurement. When peroxidase is used as the peroxidative substance, its concentration is preferably 1 to 100 kU/L. The concentration of an oxidative coloring type chromogen is not specifically limited as long as it is suited for the measurement, but it is preferably 0.01 to 10 g/L.

In the present invention, it is preferred that the measurement of RLP-C is carried out in an aqueous medium. Examples of the aqueous media include deionized water, distilled water and a buffer solution, and preferred is a buffer solution. Examples of the buffers used in the buffer solution are tris(hydroxymethyl)aminomethane buffer, phosphate buffer, borate buffer and Good's buffer.

Examples of Good's buffer include 2-morpholinoethanesulfonic acid (MES), bis(2-hydroxyethyl)iminotris(hydroxymethyl)methane (Bis-Tris), N-(2-acetamido)iminodiacetic acid (ADA), piperazine-N,N'-bis(2-ethanesulfonic acid) (PIPES), N-(2-acetamido)-2-aminoethanesulfonic acid (ACES), 3-morpholino-2-hydroxypropanesulfonic acid (MOPSO), N,N-bis(2-hydroxyethyl)-2-aminoethanesulfonic acid (BES), 3-morpholinopropanesulfonic acid (MOPS), N-[tris(hydroxymethyl)methyl]-2-aminoethanesulfonic acid (TES), 2-[4-(2-hydroxyethyl)-1-piperazinyl]ethanesulfonic acid (HEPES), 3-[N,N-bis(2-hydroxyethyl)amino]-2-hydroxypropanesulfonic acid (DIPSO), N-[tris(hydroxymethyl)methyl]-2-hydroxy-3-aminopropanesulfonic acid (TAPSO), piperazine-N,N'-bis(2-hydroxypropanesulfonic acid) (POPSO), 3-[4-(2-hydroxyethyl)-1-piperazinyl]-2-hydroxypropanesulfonic acid (HEPPSO), 3-[4-(2-hydroxyethyl)-1-piperazinyl]propanesulfonic acid [(H)EPPS], N-[tris(hydroxymethyl)methyl]glycine (Tricine), N,N-bis(2-hydroxyethyl)glycine (Bicine), N-tris(hydroxymethyl)methyl-3-aminopropanesulfonic acid (TAPS), N-cyclohexyl-2-aminoethanesulfonic acid (CHES), N-cyclohexyl-3-amino-2-hydroxypropanesulfonic acid (CAPSO) and N-cyclohexyl-3-aminopropanesulfonic acid (CAPS). The concentration of the buffer solution is not specifically limited as long as it is suited for the measurement, but it is preferably 0.001 to 2.0 mol/L, more preferably 0.005 to 1.0 mol/L.

The method, reagent and kit for the measurement of RLP-C of the present invention are specifically described below.

### (Method for the Measurement of RLP-C)

The method for the measurement of RLP-C of the present invention comprises the steps of: (i) eliminating cholesterol in lipoproteins other than RLP by reacting a sample with cholesterol oxidase, or cholesterol dehydrogenase and oxidized coenzyme, in an aqueous medium comprising surfactant A; (ii) reacting the reaction solution from which cholesterol in lipoproteins other than RLP has been eliminated in the above step (i) with cholesterol esterase and cholesterol oxidase, or cholesterol esterase, cholesterol dehydrogenase and oxidized coenzyme, in the presence of surfactant B to form hydrogen peroxide or reduced coenzyme; and (iii) measuring the hydrogen peroxide or reduced coenzyme formed in the above step (ii).

In the method of the present invention, step (i) can be carried out in the presence of a polyanion, a polyanion and a divalent metal ion, or an antibody selected from the group consisting of anti-apoB antibodies and anti-apoE antibodies. In the presence of a polyanion, surfactant (A1) is preferably used as surfactant A. In the presence of a polyanion and a divalent metal ion, or an antibody selected from the group consisting of anti-apoB antibodies and anti-apoE antibodies, surfactant (A2) is preferably used as surfactant A.

An example of the step of eliminating cholesterol in lipoproteins other than RLP is a step of eliminating hydrogen peroxide or reduced coenzyme formed in step (i) of the present invention.

The step of eliminating hydrogen peroxide formed in step (i) of the present invention can be carried out, for example, by a method in which catalase is allowed to be present in step (i) and a method in which peroxidase and one of the two oxidative coupling-type chromogens used for oxidative coupling coloring reaction are used.

The step of eliminating reduced coenzyme formed in step (i) of the present invention can be carried out, for example, by a method which comprises allowing reduced coenzyme oxidase to act on reduced coenzyme to form hydrogen peroxide in step (i), and then eliminating hydrogen peroxide in the same manner as above.

In the present invention, the step of eliminating cholesterol in lipoproteins other than RLP in step (i) is not limited to a step in which hydrogen peroxide or reduced coenzyme formed from the cholesterol is converted into another substance and eliminated. When hydrogen peroxide and reduced coenzyme can be measured separately, hydrogen peroxide or reduced coenzyme formed from the cholesterol is not required to be converted into another substance. For example, when reduced coenzyme is formed from cholesterol in lipoproteins other than RLP in step (i) and hydrogen peroxide is formed from cholesterol in RLP in step (ii), the reduced coenzyme formed in step (i) does not need to be converted into another substance. Further, the present invention includes the measurement of RLP-C in a sample by calculating the amount of hydrogen peroxide or reduced coenzyme formed in step (ii) based on the measurement value obtained by measuring hydrogen peroxide or reduced coenzyme formed in step (i).

Certain embodiments of the method for the measurement of RLP-C of the present invention are illustrated below.

### Method for the measurement

(1) The concentration of RLP-C in a sample is calculated by: reacting the sample with cholesterol oxidase, or cholesterol oxidase and cholesterol esterase, in an aqueous medium comprising surfactant A and catalase; eliminating the formed hydrogen peroxide; reacting the resulting reaction solution with a group of enzymes consisting of cholesterol oxidase and cholesterol esterase and, if necessary, further with a phospholipid-hydrolyzing enzyme in an aqueous medium comprising surfactant B and a catalase inhibitor to form hydrogen peroxide; and measuring the formed hydrogen peroxide.
(2) The concentration of RLP-C in a sample is calculated by: reacting the sample with a group of enzymes consisting of cholesterol dehydrogenase, oxidized coenzyme and reduced coenzyme oxidase, or a group of enzymes consisting of cholesterol esterase, cholesterol dehydrogenase, oxidized coenzyme and reduced coenzyme oxidase, in an aqueous medium comprising surfactant A and catalase; eliminating the formed hydrogen peroxide; reacting the resulting reaction solution with a group of enzymes consisting of cholesterol esterase, cholesterol dehydrogenase, oxidized coenzyme and reduced coenzyme oxidase and, if necessary, further with a phospholipid-hydrolyzing enzyme in an aqueous medium comprising surfactant B and a catalase inhibitor to form hydrogen peroxide; and measuring the formed hydrogen peroxide.
(3) The concentration of RLP-C in a sample is calculated by: reacting the sample with a group of enzymes consisting of cholesterol oxidase, cholesterol dehydrogenase, oxidized coenzyme and reduced coenzyme oxidase, or a group of enzymes consisting of cholesterol esterase, cholesterol oxidase, cholesterol dehydrogenase, oxidized coenzyme and reduced coenzyme oxidase, in an aqueous medium comprising surfactant A and catalase; eliminating the formed hydrogen peroxide; reacting the resulting reaction solution with a group of enzymes consisting of cholesterol esterase, cholesterol oxidase, cholesterol dehydrogenase, oxidized coenzyme and reduced coenzyme oxidase and, if necessary, further with a phospholipid-hydrolyzing enzyme in an aqueous medium comprising surfactant B and a catalase inhibitor to form hydrogen peroxide; and measuring the formed hydrogen peroxide.
(4) The concentration of RLP-C in a sample is calculated by: reacting the sample with cholesterol oxidase, or cholesterol oxidase and cholesterol esterase, in an aqueous medium comprising surfactant A and a combination of peroxidase and one of the two oxidative coupling-type chromogens used for oxidative coloring reaction; eliminating the formed hydrogen peroxide; reacting the resulting reaction solution with a group of enzymes consisting of cholesterol oxidase and cholesterol esterase and, if necessary, further with a phospholipid-hydrolyzing enzyme in an aqueous medium comprising surfactant B and the other oxidative coupling-type chromogen to form hydrogen peroxide; and measuring the formed hydrogen peroxide.
(5) The concentration of RLP-C in a sample is calculated by: reacting the sample with a group of enzymes consisting of cholesterol dehydrogenase, oxidized coenzyme and reduced coenzyme oxidase, or a group of enzymes consisting of cholesterol esterase, cholesterol dehydrogenase, oxidized coenzyme and reduced coenzyme oxidase, in an aqueous medium comprising surfactant A and a combination of peroxidase and one of the two oxidative coupling-type chromogens used for oxidative coloring reaction; eliminating the formed hydrogen peroxide; reacting the resulting reaction solution with a group of enzymes consisting of cholesterol esterase, cholesterol dehydrogenase, oxidized coenzyme and reduced coenzyme oxidase and, if necessary, further with a phospholipid-hydrolyzing enzyme in an aqueous medium comprising surfactant B and the other oxidative coupling-type chromogen to form hydrogen peroxide; and measuring the formed hydrogen peroxide.
(6) The concentration of RLP-C in a sample is calculated by: reacting the sample with a group of enzymes consisting of cholesterol oxidase, cholesterol dehydrogenase, oxidized coenzyme and reduced coenzyme oxidase, or a group of enzymes consisting of cholesterol esterase, cholesterol oxidase, cholesterol dehydrogenase, oxidized coenzyme and reduced coenzyme oxidase, in an aqueous medium comprising surfactant A and a combination of peroxidase and one of the two oxidative coupling-type chromogens used for oxidative coloring reaction; eliminating the formed hydrogen peroxide; reacting the resulting reaction solution with a group of enzymes consisting of cholesterol esterase, cholesterol oxidase, cholesterol dehydrogenase, oxidized coenzyme and reduced coenzyme oxidase and, if necessary, further with a phospholipid-hydrolyzing enzyme in an aqueous medium comprising surfactant B and the other oxidative coupling-type chromogen to form hydrogen peroxide; and measuring the formed hydrogen peroxide.
(7) The concentration of RLP-C in a sample is calculated by: reacting the sample with cholesterol oxidase, or cholesterol oxidase and cholesterol esterase, in an aqueous medium comprising surfactant (A1), a polyanion and catalase; eliminating the formed hydrogen peroxide; reacting the resulting reaction solution with a group of enzymes consisting of cholesterol oxidase and cholesterol esterase and, if necessary, further with a phospholipid-hydrolyzing enzyme in an aqueous medium comprising surfactant B and a catalase inhibitor to form hydrogen peroxide; and measuring the formed hydrogen peroxide.
(8) The concentration of RLP-C in a sample is calculated by: reacting the sample with a group of enzymes consisting of cholesterol dehydrogenase, oxidized coenzyme and reduced coenzyme oxidase, or a group of enzymes consisting of cholesterol esterase, cholesterol dehydrogenase, oxidized coenzyme and reduced coenzyme oxidase, in an aqueous medium comprising surfactant (A1), a polyanion and catalase; eliminating the formed hydrogen peroxide; reacting the resulting reaction solution with a group of enzymes consisting of cholesterol esterase, cholesterol dehydrogenase, oxidized coenzyme and reduced coenzyme oxidase and, if necessary, further with a phospholipid-hydrolyzing enzyme in an aqueous medium comprising surfactant B and a catalase inhibitor to form hydrogen peroxide; and measuring the formed hydrogen peroxide.
(9) The concentration of RLP-C in a sample is calculated by: reacting the sample with a group of enzymes consisting of cholesterol oxidase, cholesterol dehydrogenase, oxidized coenzyme and reduced coenzyme oxidase, or a group of enzymes consisting of cholesterol esterase, cholesterol oxidase, cholesterol dehydrogenase, oxidized coenzyme and reduced coenzyme oxidase, in an aqueous medium comprising surfactant (A1), a polyanion and catalase; eliminating the formed hydrogen peroxide; reacting the resulting reaction solution with a group of enzymes consisting of cholesterol esterase, cholesterol oxidase, cholesterol dehydrogenase, oxidized coenzyme and reduced coenzyme oxidase and, if necessary, further with a phospholipid-hydrolyzing enzyme in an aqueous medium comprising surfactant B and a catalase inhibitor to form hydrogen peroxide; and measuring the formed hydrogen peroxide.
(10) The concentration of RLP-C in a sample is calculated by: reacting the sample with cholesterol oxidase, or cholesterol oxidase and cholesterol esterase, in an aqueous medium comprising surfactant (A1), a polyanion and a combination of peroxidase and one of the two oxidative coupling-type chromogens used for oxidative coloring reaction; eliminating the formed hydrogen peroxide; reacting the resulting reaction solution with a group of enzymes consisting of cholesterol oxidase and cholesterol esterase and, if necessary, further with a phospholipid-hydrolyzing enzyme in an aqueous medium comprising surfactant B and the other oxidative coupling-type chromogen to form hydrogen peroxide; and measuring the formed hydrogen peroxide.
(11) The concentration of RLP-C in a sample is calculated by: reacting the sample with a group of enzymes consisting of cholesterol dehydrogenase, oxidized coenzyme and reduced coenzyme oxidase, or a group of enzymes consisting of cholesterol esterase, cholesterol dehydrogenase, oxidized coenzyme and reduced coenzyme oxidase, in an aqueous medium comprising surfactant (A1), a polyanion and a combination of peroxidase and one of the two oxidative coupling-type chromogens used for oxidative coloring reaction; eliminating the formed hydrogen peroxide; reacting the resulting reaction solution with a group of enzymes consisting of cholesterol esterase, cholesterol dehydrogenase, oxidized coenzyme and reduced coenzyme oxidase and, if necessary, further with a phospholipid-hydrolyzing enzyme in an aqueous medium comprising surfactant B and the other oxidative coupling-type chromogen to form hydrogen peroxide; and measuring the formed hydrogen peroxide.
(12) The concentration of RLP-C in a sample is calculated by: reacting the sample with a group of enzymes consisting of cholesterol oxidase, cholesterol dehydrogenase, oxidized coenzyme and reduced coenzyme oxidase, or a group of enzymes consisting of cholesterol esterase, cholesterol oxidase, cholesterol dehydrogenase, oxidized coenzyme and reduced coenzyme oxidase, in an aqueous medium comprising surfactant (A1), a polyanion and a combination of peroxidase and one of the two oxidative coupling-type chromogens used for oxidative coloring reaction; eliminating the formed hydrogen peroxide; reacting the resulting reaction solution with a group of enzymes consisting of cholesterol esterase, cholesterol oxidase, cholesterol dehydrogenase, oxidized coenzyme and reduced coenzyme oxidase and, if necessary, further with a phospholipid-hydrolyzing enzyme in an aqueous medium comprising surfactant B and the other oxidative coupling-type chromogen to form hydrogen peroxide; and measuring the formed hydrogen peroxide.
(13) The concentration of RLP-C in a sample is calculated by: reacting the sample with cholesterol oxidase, or cholesterol oxidase and cholesterol esterase, in an aqueous medium comprising surfactant (A2), a polyanion, a divalent metal ion and catalase; eliminating the formed hydrogen peroxide; reacting the resulting reaction solution with a group of enzymes consisting of cholesterol oxidase and cholesterol esterase and, if necessary, further with a phospholipid-hydrolyzing enzyme in an aqueous medium comprising surfactant B and a catalase inhibitor to form hydrogen peroxide; and measuring the formed hydrogen peroxide.
(14) The concentration of RLP-C in a sample is calculated by: reacting the sample with cholesterol oxidase, or cholesterol oxidase and cholesterol esterase, in an aqueous medium comprising surfactant (A2), an anti-apoB antibody and/or an anti-apoE antibody, and catalase; eliminating the formed hydrogen peroxide; reacting the resulting reaction solution with a group of enzymes consisting of cholesterol oxidase and cholesterol esterase and, if necessary, further with a phospholipid-hydrolyzing enzyme in an aqueous medium comprising surfactant B and a catalase inhibitor to form hydrogen peroxide; and measuring the formed hydrogen peroxide.
(15) The concentration of RLP-C in a sample is calculated by: reacting the sample with a group of enzymes consisting of cholesterol dehydrogenase, oxidized coenzyme and reduced coenzyme oxidase, or a group of enzymes consisting of cholesterol esterase, cholesterol dehydrogenase, oxidized coenzyme and reduced coenzyme oxidase, in an aqueous medium comprising surfactant (A2), a polyanion, a divalent metal ion and catalase; eliminating the formed hydrogen peroxide; reacting the resulting reaction solution with a group of enzymes consisting of cholesterol esterase, cholesterol dehydrogenase, oxidized coenzyme and reduced coenzyme oxidase and, if necessary, further with a phospholipid-hydrolyzing enzyme in an aqueous medium comprising surfactant B and a catalase inhibitor to form hydrogen peroxide; and measuring the formed hydrogen peroxide.
(16) The concentration of RLP-C in a sample is calculated by: reacting the sample with a group of enzymes consisting of cholesterol dehydrogenase, oxidized coenzyme and reduced coenzyme oxidase, or a group of enzymes consisting of cholesterol esterase, cholesterol dehydrogenase, oxidized coenzyme and reduced coenzyme oxidase, in an aqueous medium comprising surfactant (A2), an anti-apoB antibody and/or an anti-apoE antibody, and catalase; eliminating the formed hydrogen peroxide; reacting the resulting reaction solution with a group of enzymes consisting of cholesterol esterase, cholesterol dehydrogenase, oxidized coenzyme and reduced coenzyme oxidase and, if necessary, further with a phospholipid-hydrolyzing enzyme in an aqueous medium comprising surfactant B and a catalase inhibitor to form hydrogen peroxide; and measuring the formed hydrogen peroxide.
(17) The concentration of RLP-C in a sample is calculated by: reacting the sample with a group of enzymes consisting of cholesterol oxidase, cholesterol dehydrogenase, oxidized coenzyme and reduced coenzyme oxidase, or a group of enzymes consisting of cholesterol esterase, cholesterol oxidase, cholesterol dehydrogenase, oxidized coenzyme and reduced coenzyme oxidase, in an aqueous medium comprising surfactant (A2), a polyanion, a divalent metal ion and catalase; eliminating the formed hydrogen peroxide; reacting the resulting reaction solution with a group of enzymes consisting of cholesterol esterase, cholesterol oxidase, cholesterol dehydrogenase, oxidized coenzyme and reduced coenzyme oxidase and, if necessary, further with a phospholipid-hydrolyzing enzyme in an aqueous medium comprising surfactant B and a catalase inhibitor to form hydrogen peroxide; and measuring the formed hydrogen peroxide.
(18) The concentration of RLP-C in a sample is calculated by: reacting the sample with a group of enzymes consisting of cholesterol oxidase, cholesterol dehydrogenase, oxidized coenzyme and reduced coenzyme oxidase, or a group of enzymes consisting of cholesterol esterase, cholesterol oxidase, cholesterol dehydrogenase, oxidized coenzyme and reduced coenzyme oxidase, in an aqueous medium comprising surfactant (A2), an anti-apoB antibody and/or an anti-apoE antibody, and catalase; eliminating the formed hydrogen peroxide; reacting the resulting reaction solution with a group of enzymes consisting of cholesterol esterase, cholesterol oxidase, cholesterol dehydrogenase, oxidized coenzyme and reduced coenzyme oxidase and, if necessary, further with a phospholipid-hydrolyzing enzyme in an aqueous medium comprising surfactant B and a catalase inhibitor to form hydrogen peroxide; and measuring the formed hydrogen peroxide.
(19) The concentration of RLP-C in a sample is calculated by: reacting the sample with cholesterol oxidase, or cholesterol oxidase and cholesterol esterase, in an aqueous medium comprising surfactant (A2), a polyanion, a divalent metal ion and a combination of peroxidase and one of the two oxidative coupling-type chromogens used for oxidative coloring reaction; eliminating the formed hydrogen peroxide; reacting the resulting reaction solution with a group of enzymes consisting of cholesterol oxidase and cholesterol esterase and, if necessary, further with a phospholipid-hydrolyzing enzyme in an aqueous medium comprising surfactant B and the other oxidative coupling-type chromogen to form hydrogen peroxide; and measuring the formed hydrogen peroxide.
(20) The concentration of RLP-C in a sample is calculated by: reacting the sample with cholesterol oxidase, or cholesterol oxidase and cholesterol esterase, in an aqueous medium comprising surfactant (A2), an anti-apoB antibody and/or an anti-apoE antibody, and a combination of peroxidase and one of the two oxidative coupling-type chromogens used for oxidative coloring reaction; eliminating the formed hydrogen peroxide; reacting the resulting reaction solution with a group of enzymes consisting of cholesterol oxidase and cholesterol esterase and, if necessary, further with a phospholipid-hydrolyzing enzyme in an aqueous medium comprising surfactant B and the other oxidative coupling-type chromogen to form hydrogen peroxide; and measuring the formed hydrogen peroxide.
(21) The concentration of RLP-C in a sample is calculated by: reacting the sample with a group of enzymes consisting of cholesterol dehydrogenase, oxidized coenzyme and reduced coenzyme oxidase, or a group of enzymes consisting of cholesterol esterase, cholesterol dehydrogenase, oxidized coenzyme and reduced coenzyme oxidase, in an aqueous medium comprising surfactant (A2), a polyanion, a divalent metal ion and a combination of peroxidase and one of the two oxidative coupling-type chromogens used for oxidative coloring reaction; eliminating the formed hydrogen peroxide; reacting the resulting reaction solution with a group of enzymes consisting of cholesterol esterase, cholesterol dehydrogenase, oxidized coenzyme and reduced coenzyme oxidase and, if necessary, further with a phospholipid-hydrolyzing enzyme in an aqueous medium comprising surfactant B and the other oxidative coupling-type chromogen to form hydrogen peroxide; and measuring the formed hydrogen peroxide.
(22) The concentration of RLP-C in a sample is calculated by: reacting the sample with a group of enzymes consisting of cholesterol dehydrogenase, oxidized coenzyme and reduced coenzyme oxidase, or a group of enzymes consisting of cholesterol esterase, cholesterol dehydrogenase, oxidized coenzyme and reduced coenzyme oxidase, in an aqueous medium comprising surfactant (A2), an anti-apoB antibody and/or an anti-apoE antibody, and a combination of peroxidase and one of the two oxidative coupling-type chromogens used for oxidative coloring reaction; eliminating the formed hydrogen peroxide; reacting the resulting reaction solution with a group of enzymes consisting of cholesterol esterase, cholesterol dehydrogenase, oxidized coenzyme and reduced coenzyme oxidase and, if necessary, further with a phospholipid-hydrolyzing enzyme in an aqueous medium comprising surfactant B and the other oxidative coupling-type chromogen to form hydrogen peroxide; and measuring the formed hydrogen peroxide.
(23) The concentration of RLP-C in a sample is calculated by: reacting the sample with a group of enzymes consisting of cholesterol oxidase, cholesterol dehydrogenase, oxidized coenzyme and reduced coenzyme oxidase, or a group of enzymes consisting of cholesterol esterase, cholesterol oxidase, cholesterol dehydrogenase, oxidized coenzyme and reduced coenzyme oxidase, in an aqueous medium comprising surfactant (A2), a polyanion, a divalent metal ion and a combination of peroxidase and one of the two oxidative coupling-type chromogens used for oxidative coloring reaction; eliminating the formed hydrogen peroxide; reacting the resulting reaction solution with a group of enzymes consisting of cholesterol esterase, cholesterol oxidase, cholesterol dehydrogenase, oxidized coenzyme and reduced coenzyme oxidase and, if necessary, further with a phospholipid-hydrolyzing enzyme in an aqueous medium comprising surfactant B and the other oxidative coupling-type chromogen to form hydrogen peroxide; and measuring the formed hydrogen peroxide.
(24) The concentration of RLP-C in a sample is calculated by: reacting the sample with a group of enzymes consisting of cholesterol oxidase, cholesterol dehydrogenase, oxidized coenzyme and reduced coenzyme oxidase, or a group of enzymes consisting of cholesterol esterase, cholesterol oxidase, cholesterol dehydrogenase, oxidized coenzyme and reduced coenzyme oxidase, in an aqueous medium comprising surfactant (A2), an anti-apoB antibody and/or an anti-apoE antibody, and a combination of peroxidase and one of the two oxidative coupling-type chromogens used for oxidative coloring reaction; eliminating the formed hydrogen peroxide; reacting the resulting reaction solution with a group of enzymes consisting of cholesterol esterase, cholesterol oxidase, cholesterol dehydrogenase, oxidized coenzyme and reduced coenzyme oxidase and, if necessary, further with a phospholipid-hydrolyzing enzyme in an aqueous medium comprising surfactant B and the other oxidative coupling-type chromogen to form hydrogen peroxide; and measuring the formed hydrogen peroxide.

The reaction in the present method for the measurement is carried out, for example, at 10 to 50°C, preferably 20 to 40°C for 1 to 60 minutes, preferably 2 to 30 minutes. The amount of the formed hydrogen peroxide can be measured, for example, directly with a hydrogen peroxide electrode, and also by using a reagent for the measurement of hydrogen peroxide.

### (Reagent for the Measurement of RLP-C)

The reagent for the measurement of RLP-C of the present invention can be used for the measurement of RLP-C of the present invention.

An example of the reagent for the measurement of RLP-C of the present invention is the following.

A reagent for the measurement of RLP-C in a sample comprising cholesterol esterase, cholesterol oxidase, a reagent for the elimination of hydrogen peroxide, a reagent for the measurement of hydrogen peroxide, surfactant A and surfactant B.

Examples of the reagent for the elimination of hydrogen peroxide are a reagent comprising peroxidase and one of the two oxidative coupling-type chromogens used for oxidative coupling coloring reaction, and a reagent comprising catalase.

Another example of the reagent for the measurement of RLP-C of the present invention is the following.

A reagent for the measurement of RLP-C in a sample comprising cholesterol esterase, cholesterol dehydrogenase, oxidized coenzyme, surfactant A and surfactant B.

Further, another example of the reagent for the measurement of RLP-C of the present invention is the following.

A reagent for the measurement of RLP-C in a sample comprising cholesterol esterase, cholesterol dehydrogenase, oxidized coenzyme, reduced coenzyme oxidase, a reagent for the elimination of hydrogen peroxide, a reagent for the measurement of hydrogen peroxide, surfactant A and surfactant B.

Examples of the reagent for the elimination of hydrogen peroxide are a reagent comprising peroxidase and one of the two oxidative coupling-type chromogens used for oxidative coupling coloring reaction, and a reagent comprising catalase.

These reagents may further comprise a polyanion, and in that case, surfactant (A1) is preferably used as surfactant A.

These reagents may also comprise a polyanion and a divalent metal ion, or an antibody selected from the group consisting of anti-apoB antibodies and anti-apoE antibodies, and in that case, surfactant (A2) is preferably used as surfactant A.

These reagents may further comprise a phospholipid-hydrolyzing enzyme.

Certain specific embodiments of the reagent for the measurement of RLP-C of the present invention are illustrated below, but they are not to be construed as limiting the scope of the present invention. In the present invention, as surfactant A, surfactant (A1), surfactant (A2) and surfactant B, one or plural kinds of surfactants selected from the respective groups can be used.

### Reagent 1

A reagent comprising cholesterol esterase, cholesterol oxidase, catalase, a catalase inhibitor, surfactant A and surfactant B

### Reagent 2

A reagent comprising cholesterol esterase, cholesterol oxidase, catalase, a catalase inhibitor, surfactant A, surfactant B and a reagent for the measurement of hydrogen peroxide

### Reagent 3

A reagent comprising cholesterol esterase, cholesterol dehydrogenase, reduced coenzyme oxidase, oxidized coenzyme, catalase, a catalase inhibitor, surfactant A and surfactant B

### Reagent 4

A reagent comprising cholesterol esterase, cholesterol dehydrogenase, reduced coenzyme oxidase, oxidized coenzyme, catalase, a catalase inhibitor, surfactant A, surfactant B and a reagent for the measurement of hydrogen peroxide

### Reagent 5

A reagent comprising cholesterol esterase, cholesterol oxidase, cholesterol dehydrogenase, reduced coenzyme oxidase, oxidized coenzyme, catalase, a catalase inhibitor, surfactant A and surfactant B

### Reagent 6

A reagent comprising cholesterol esterase, cholesterol oxidase, cholesterol dehydrogenase, reduced coenzyme oxidase, oxidized coenzyme, catalase, a catalase inhibitor, surfactant A, surfactant B and a reagent for the measurement of hydrogen peroxide

### Reagent 7

A reagent comprising cholesterol esterase, cholesterol oxidase, peroxidase, oxidative coupling-type chromogens, surfactant A and surfactant B

### Reagent 8

A reagent comprising cholesterol esterase, cholesterol dehydrogenase, reduced coenzyme oxidase, oxidized coenzyme, peroxidase, oxidative coupling-type chromogens, surfactant A and surfactant B

### Reagent 9

A reagent comprising cholesterol esterase, cholesterol oxidase, cholesterol dehydrogenase, reduced coenzyme oxidase, oxidized coenzyme, peroxidase, oxidative coupling-type chromogens, surfactant A and surfactant B

### Reagent 10

A reagent comprising cholesterol esterase, cholesterol oxidase, a phospholipid-hydrolyzing enzyme, catalase, a catalase inhibitor, surfactant A and surfactant B

### Reagent 11

A reagent comprising cholesterol esterase, cholesterol oxidase, a phospholipid-hydrolyzing enzyme, catalase, a catalase inhibitor, surfactant A, surfactant B and a reagent for the measurement of hydrogen peroxide

### Reagent 12

A reagent comprising cholesterol esterase, cholesterol dehydrogenase, reduced coenzyme oxidase, oxidized coenzyme, a phospholipid-hydrolyzing enzyme, catalase, a catalase inhibitor, surfactant A and surfactant B

### Reagent 13

A reagent comprising cholesterol esterase, cholesterol dehydrogenase, reduced coenzyme oxidase, oxidized coenzyme, a phospholipid-hydrolyzing enzyme, catalase, a catalase inhibitor, surfactant A, surfactant B and a reagent for the measurement of hydrogen peroxide

### Reagent 14

A reagent comprising cholesterol esterase, cholesterol oxidase, cholesterol dehydrogenase, reduced coenzyme oxidase, oxidized coenzyme, a phospholipid-hydrolyzing enzyme, catalase, a catalase inhibitor, surfactant A and surfactant B

### Reagent 15

A reagent comprising cholesterol esterase, cholesterol oxidase, cholesterol dehydrogenase, reduced coenzyme oxidase, oxidized coenzyme, a phospholipid-hydrolyzing enzyme, catalase, a catalase inhibitor, surfactant A, surfactant B and a reagent for the measurement of hydrogen peroxide

### Reagent 16

A reagent comprising cholesterol esterase, cholesterol oxidase, a phospholipid-hydrolyzing enzyme, peroxidase, oxidative coupling-type chromogens, surfactant A and surfactant B

### Reagent 17

A reagent comprising cholesterol esterase, cholesterol dehydrogenase, reduced coenzyme oxidase, oxidized coenzyme, a phospholipid-hydrolyzing enzyme, peroxidase, oxidative coupling-type chromogens, surfactant A and surfactant B

### Reagent 18

A reagent comprising cholesterol esterase, cholesterol oxidase, cholesterol dehydrogenase, reduced coenzyme oxidase, oxidized coenzyme, a phospholipid-hydrolyzing enzyme, peroxidase, oxidative coupling-type chromogens, surfactant A and surfactant B

### Reagent 19

A reagent comprising cholesterol esterase, cholesterol oxidase, a polyanion, catalase, a catalase inhibitor, surfactant (A1) and surfactant B

### Reagent 20

A reagent comprising cholesterol esterase, cholesterol oxidase, a polyanion, catalase, a catalase inhibitor, surfactant (A1), surfactant B and a reagent for the measurement of hydrogen peroxide

### Reagent 21

A reagent comprising cholesterol esterase, cholesterol dehydrogenase, reduced coenzyme oxidase, oxidized coenzyme, a polyanion, catalase, a catalase inhibitor, surfactant (A1) and surfactant B

### Reagent 22

A reagent comprising cholesterol esterase, cholesterol dehydrogenase, reduced coenzyme oxidase, oxidized coenzyme, a polyanion, catalase, a catalase inhibitor, surfactant (A1), surfactant B and a reagent for the measurement of hydrogen peroxide

### Reagent 23

A reagent comprising cholesterol esterase, cholesterol oxidase, cholesterol dehydrogenase, reduced coenzyme oxidase, oxidized coenzyme, a polyanion, catalase, a catalase inhibitor, surfactant (A1) and surfactant B

### Reagent 24

A reagent comprising cholesterol esterase, cholesterol oxidase, cholesterol dehydrogenase, reduced coenzyme oxidase, oxidized coenzyme, a polyanion, catalase, a catalase inhibitor, surfactant (A1), surfactant B and a reagent for the measurement of hydrogen peroxide

### Reagent 25

A reagent comprising cholesterol esterase, cholesterol oxidase, a polyanion, peroxidase, oxidative coupling-type chromogens, surfactant (A1) and surfactant B

### Reagent 26

A reagent comprising cholesterol esterase, cholesterol dehydrogenase, reduced coenzyme oxidase, oxidized coenzyme, a polyanion, peroxidase, oxidative coupling-type chromogens, surfactant (A1) and surfactant B

### Reagent 27

A reagent comprising cholesterol esterase, cholesterol oxidase, cholesterol dehydrogenase, reduced coenzyme oxidase, oxidized coenzyme, a polyanion, peroxidase, oxidative coupling-type chromogens, surfactant (A1) and surfactant B

### Reagent 28

A reagent comprising cholesterol esterase, cholesterol oxidase, a phospholipid-hydrolyzing enzyme, a polyanion, catalase, a catalase inhibitor, surfactant (A1) and surfactant B

### Reagent 29

A reagent comprising cholesterol esterase, cholesterol oxidase, a phospholipid-hydrolyzing enzyme, a polyanion, catalase, a catalase inhibitor, surfactant (d1), surfactant B and a reagent for the measurement of hydrogen peroxide

### Reagent 30

A reagent comprising cholesterol esterase, cholesterol dehydrogenase, reduced coenzyme oxidase, oxidized coenzyme, a phospholipid-hydrolyzing enzyme, a polyanion, catalase, a catalase inhibitor, surfactant (A1) and surfactant B

### Reagent 31

A reagent comprising cholesterol esterase, cholesterol dehydrogenase, reduced coenzyme oxidase, oxidized coenzyme, a phospholipid-hydrolyzing enzyme, a polyanion, catalase, a catalase inhibitor, surfactant (A1), surfactant B and a reagent for the measurement of hydrogen peroxide

### Reagent 32

A reagent comprising cholesterol esterase, cholesterol oxidase, cholesterol dehydrogenase, reduced coenzyme oxidase, oxidized coenzyme, a phospholipid-hydrolyzing enzyme, a polyanion, catalase, a catalase inhibitor, surfactant (A1) and surfactant B

### Reagent 33

A reagent comprising cholesterol esterase, cholesterol oxidase, cholesterol dehydrogenase, reduced coenzyme oxidase, oxidized coenzyme, a phospholipid-hydrolyzing enzyme, a polyanion, catalase, a catalase inhibitor, surfactant (A1), surfactant B and a reagent for the measurement of hydrogen peroxide

### Reagent 34

A reagent comprising cholesterol esterase, cholesterol oxidase, a phospholipid-hydrolyzing enzyme, a polyanion, peroxidase, oxidative coupling-type chromogens, surfactant (A1) and surfactant B

### Reagent 35

A reagent comprising cholesterol esterase, cholesterol dehydrogenase, reduced coenzyme oxidase, oxidized coenzyme, a phospholipid-hydrolyzing enzyme, a polyanion, peroxidase, oxidative coupling-type chromogens, surfactant (A1) and surfactant B

### Reagent 36

A reagent comprising cholesterol esterase, cholesterol oxidase, cholesterol dehydrogenase, reduced coenzyme oxidase, oxidized coenzyme, a phospholipid-hydrolyzing enzyme, a polyanion, peroxidase, oxidative coupling-type chromogens, surfactant (A1) and surfactant B

### Reagent 37

A reagent comprising cholesterol esterase, cholesterol oxidase, a polyanion, a divalent metal ion, catalase, a catalase inhibitor, surfactant (A2) and surfactant B

### Reagent 38

A reagent comprising cholesterol esterase, cholesterol oxidase, a polyanion, a divalent metal ion, catalase, a catalase inhibitor, surfactant (A2), surfactant B and a reagent for the measurement of hydrogen peroxide

### Reagent 39

A reagent comprising cholesterol esterase, cholesterol oxidase, an anti-apoB antibody and/or an anti-apoE antibody, catalase, a catalase inhibitor, surfactant (A2) and surfactant B

### Reagent 40

A reagent comprising cholesterol esterase, cholesterol oxidase, an anti-apoB antibody and/or an anti-apoE antibody, catalase, a catalase inhibitor, surfactant (A2), surfactant B and a reagent for the measurement of hydrogen peroxide

### Reagent 41

A reagent comprising cholesterol esterase, cholesterol dehydrogenase, reduced coenzyme oxidase, oxidized coenzyme, a polyanion, a divalent metal ion, catalase, a catalase inhibitor, surfactant (A2) and surfactant B

### Reagent 42

A reagent comprising cholesterol esterase, cholesterol dehydrogenase, reduced coenzyme oxidase, oxidized coenzyme, a polyanion, a divalent metal ion, catalase, a catalase inhibitor, surfactant (A2), surfactant B and a reagent for the measurement of hydrogen peroxide

### Reagent 43

A reagent comprising cholesterol esterase, cholesterol dehydrogenase, reduced coenzyme oxidase, oxidized coenzyme, an anti-apoB antibody and/or an anti-apoE antibody, catalase, a catalase inhibitor, surfactant (A2) and surfactant B

### Reagent 44

A reagent comprising cholesterol esterase, cholesterol dehydrogenase, reduced coenzyme oxidase, oxidized coenzyme, an anti-apoB antibody and/or an anti-apoE antibody, catalase, a catalase inhibitor, surfactant (A2), surfactant B and a reagent for the measurement of hydrogen peroxide

### Reagent 45

A reagent comprising cholesterol esterase, cholesterol oxidase, cholesterol dehydrogenase, reduced coenzyme oxidase, oxidized coenzyme, a polyanion, a divalent metal ion, catalase, a catalase inhibitor, surfactant (A2) and surfactant B

### Reagent 46

A reagent comprising cholesterol esterase, cholesterol oxidase, cholesterol dehydrogenase, reduced coenzyme oxidase, oxidized coenzyme, a polyanion, a divalent metal ion, catalase, a catalase inhibitor, surfactant (A2), surfactant B and a reagent for the measurement of hydrogen peroxide

### Reagent 47

A reagent comprising cholesterol esterase, cholesterol oxidase, cholesterol dehydrogenase, reduced coenzyme oxidase, oxidized coenzyme, an anti-apoB antibody and/or an anti-apoE antibody, catalase, a catalase inhibitor, surfactant (A2) and surfactant B

### Reagent 48

A reagent comprising cholesterol esterase, cholesterol oxidase, cholesterol dehydrogenase, reduced coenzyme oxidase, oxidized coenzyme, an anti-apoB antibody and/or an anti-apoE antibody, catalase, a catalase inhibitor, surfactant (A2), surfactant B and a reagent for the measurement of hydrogen peroxide

### Reagent 49

A reagent comprising cholesterol esterase, cholesterol oxidase, a polyanion, a divalent metal ion, peroxidase, oxidative coupling-type chromogens, surfactant (A2) and surfactant B

### Reagent 50

A reagent comprising cholesterol esterase, cholesterol oxidase, an anti-apoB antibody and/or an anti-apoE antibody, peroxidase, oxidative coupling-type chromogens, surfactant (A2) and surfactant B

### Reagent 51

A reagent comprising cholesterol esterase, cholesterol dehydrogenase, reduced coenzyme oxidase, oxidized coenzyme, a polyanion, a divalent metal ion, peroxidase, oxidative coupling-type chromogens, surfactant (A2) and surfactant B

### Reagent 52

A reagent comprising cholesterol esterase, cholesterol dehydrogenase, reduced coenzyme oxidase, oxidized coenzyme, an anti-apoB antibody and/or an anti-apoE antibody, peroxidase, oxidative coupling-type chromogens, surfactant (A2) and surfactant B

### Reagent 53

A reagent comprising cholesterol esterase, cholesterol oxidase, cholesterol dehydrogenase, reduced coenzyme oxidase, oxidized coenzyme, a polyanion, a divalent metal ion, peroxidase, oxidative coupling-type chromogens, surfactant (A2) and surfactant B

### Reagent 54

A reagent comprising cholesterol esterase, cholesterol oxidase, cholesterol dehydrogenase, reduced coenzyme oxidase, oxidized coenzyme, an anti-apoB antibody and/or an anti-apoE antibody, peroxidase, oxidative coupling-type chromogens, surfactant (A2) and surfactant B

### Reagent 55

A reagent comprising cholesterol esterase, cholesterol oxidase, a phospholipid-hydrolyzing enzyme, a polyanion, a divalent metal ion, catalase, a catalase inhibitor, surfactant (A2) and surfactant B

### Reagent 56

A reagent comprising cholesterol esterase, cholesterol oxidase, a phospholipid-hydrolyzing enzyme, a polyanion, a divalent metal ion, catalase, a catalase inhibitor, surfactant (A2), surfactant B and a reagent for the measurement of hydrogen peroxide

### Reagent 57

A reagent comprising cholesterol esterase, cholesterol oxidase, a phospholipid-hydrolyzing enzyme, an anti-apoB antibody and/or an anti-apoE antibody, catalase, a catalase inhibitor, surfactant (A2) and surfactant B

### Reagent 58

A reagent comprising cholesterol esterase, cholesterol oxidase, a phospholipid-hydrolyzing enzyme, an anti-apoB antibody and/or an anti-apoE antibody, catalase, a catalase inhibitor, surfactant (A2), surfactant B and a reagent for the measurement of hydrogen peroxide

### Reagent 59

A reagent comprising cholesterol esterase, cholesterol dehydrogenase, reduced coenzyme oxidase, oxidized coenzyme, a phospholipid-hydrolyzing enzyme, a polyanion, a divalent metal ion, catalase, a catalase inhibitor, surfactant (A2) and surfactant B

### Reagent 60

A reagent comprising cholesterol esterase, cholesterol dehydrogenase, reduced coenzyme oxidase, oxidized coenzyme, a phospholipid-hydrolyzing enzyme, a polyanion, a divalent metal ion, catalase, a catalase inhibitor, surfactant (A2), surfactant B and a reagent for the measurement of hydrogen peroxide

### Reagent 61

A reagent comprising cholesterol esterase, cholesterol dehydrogenase, reduced coenzyme oxidase, oxidized coenzyme, a phospholipid-hydrolyzing enzyme, an anti-apoB antibody and/or an anti-apoA antibody, catalase, a catalase inhibitor, surfactant (A2) and surfactant B

### Reagent 62

A reagent comprising cholesterol esterase, cholesterol dehydrogenase, reduced coenzyme oxidase, oxidized coenzyme, a phospholipid-hydrolyzing enzyme, an anti-apoB antibody and/or an anti-apoE antibody, catalase, a catalase inhibitor, surfactant (A2), surfactant B and a reagent for the measurement of hydrogen peroxide

### Reagent 63

A reagent comprising cholesterol esterase, cholesterol oxidase, cholesterol dehydrogenase, reduced coenzyme oxidase, oxidized coenzyme, a phospholipid-hydrolyzing enzyme, a polyanion, a divalent metal ion, catalase, a catalase inhibitor, surfactant (A2) and surfactant B

### Reagent 64

A reagent comprising cholesterol esterase, cholesterol oxidase, cholesterol dehydrogenase, reduced coenzyme oxidase, oxidized coenzyme, a phospholipid-hydrolyzing enzyme, a polyanion, a divalent metal ion, catalase, a catalase inhibitor, surfactant (A2), surfactant B and a reagent for the measurement of hydrogen peroxide

### Reagent 65

A reagent comprising cholesterol esterase, cholesterol oxidase, cholesterol dehydrogenase, reduced coenzyme oxidase, oxidized coenzyme, a phospholipid-hydrolyzing enzyme, an anti-apoB antibody and/or an anti-apoE antibody, catalase, a catalase inhibitor, surfactant (A2) and surfactant B

### Reagent 66

A reagent comprising cholesterol esterase, cholesterol oxidase, cholesterol dehydrogenase, reduced coenzyme oxidase, oxidized coenzyme, a phospholipid-hydrolyzing enzyme, an anti-apoB antibody and/or an anti-apoE antibody, catalase, a catalase inhibitor, surfactant (A2), surfactant B and a reagent for the measurement of hydrogen peroxide

### Reagent 67

A reagent comprising cholesterol esterase, cholesterol oxidase, a phospholipid-hydrolyzing enzyme, a polyanion, a divalent metal ion, peroxidase, oxidative coupling-type chromogens, surfactant (A2) and surfactant B

### Reagent 68

A reagent comprising cholesterol esterase, cholesterol oxidase, a phospholipid-hydrolyzing enzyme, an anti-apoB antibody and/or an anti-apoE antibody, peroxidase, oxidative coupling-type chromogens, surfactant (A2) and surfactant B

### Reagent 69

A reagent comprising cholesterol esterase, cholesterol dehydrogenase, reduced coenzyme oxidase, oxidized coenzyme, a phospholipid-hydrolyzing enzyme, a polyanion, a divalent metal ion, peroxidase, oxidative coupling-type chromogens, surfactant (A2) and surfactant B

### Reagent 70

A reagent comprising cholesterol esterase, cholesterol dehydrogenase, reduced coenzyme oxidase, oxidized coenzyme, a phospholipid-hydrolyzing enzyme, an anti-apoB antibody and/or an anti-apoE antibody, peroxidase, oxidative coupling-type chromogens, surfactant (A2) and surfactant B

### Reagent 71

A reagent comprising cholesterol esterase, cholesterol oxidase, cholesterol dehydrogenase, reduced coenzyme oxidase, oxidized coenzyme, a phospholipid-hydrolyzing enzyme, a polyanion, a divalent metal ion, peroxidase, oxidative coupling-type chromogens, surfactant (A2) and surfactant B

### Reagent 72

A reagent comprising cholesterol esterase, cholesterol oxidase, cholesterol dehydrogenase, reduced coenzyme oxidase, oxidized coenzyme, a phospholipid-hydrolyzing enzyme, an anti-apoB antibody and/or an anti-apoE antibody, peroxidase, oxidative coupling-type chromogens, surfactant (A2) and surfactant B

### (Kit for the Measurement of RLP-C)

The reagent for the measurement of RLP-C of the present invention may be preserved, distributed and used in the form of a kit. There is no specific restriction as to the form of a kit so long as the measurement of RLP-C according to the present invention can be performed. A kit may be composed of two reagents or three reagents, and preferred is a kit composed of two reagents.

An example of the kit for the measurement of RLP-C of the present invention is the following.

A kit for the measurement of RLP-C which comprises a first reagent comprising cholesterol oxidase, a reagent for the elimination of hydrogen peroxide and surfactant A and a second reagent comprising a reagent for the measurement of hydrogen peroxide and surfactant B, said kit further comprising cholesterol esterase in either or both of the first reagent and the second reagent.

Another example of the kit for the measurement of RLP-C of the present invention is the following.

A kit for the measurement of RLP-C which comprises a first reagent comprising cholesterol dehydrogenase, oxidized coenzyme and surfactant A and a second reagent comprising surfactant B, said kit further comprising cholesterol esterase in either or both of the first reagent and the second reagent.

Further, another example of the kit for the measurement of RLP-C of the present invention is the following.

A kit for the measurement of RLP-C which comprises a first reagent comprising cholesterol dehydrogenase, oxidized coenzyme, reduced coenzyme oxidase, a reagent for the elimination of hydrogen peroxide and surfactant A and a second reagent comprising a reagent for the measurement of hydrogen peroxide and surfactant B, said kit further comprising cholesterol esterase in either or both of the first reagent and the second reagent.

These kits may further comprise a polyanion, and in that case, surfactant (A1) is preferably used as surfactant A.

These kits may also comprise a polyanion and a divalent metal ion, or an antibody selected from the group consisting of anti-apoB antibodies and anti-apoE antibodies, and in that case, surfactant (A2) is preferably used as surfactant A.

These kits may further comprise a phospholipid-hydrolyzing enzyme.

In the kit for the measurement of RLP-C composed of two reagents (a first reagent and a second reagent), cholesterol oxidase is contained in the first reagent, but may be contained in both of the first reagent and the second reagent.

Surfactant A, surfactant (A1) and surfactant (A2) are preferably contained in the first reagent.

Cholesterol esterase may be contained in either or both of the first reagent and the second reagent, but is preferably contained in both of the first reagent and the second reagent.

Cholesterol dehydrogenase is contained in the first reagent, but may be contained in both of the first reagent and the second reagent.

Reduced coenzyme oxidase is contained in the first reagent, but may be contained in both of the first reagent and the second reagent.

Oxidized coenzyme is contained in the first reagent, but may be contained in both of the first reagent and the second reagent.

Catalase used for the elimination of hydrogen peroxide formed in the first reaction is preferably contained in the first reagent, and a catalase inhibitor is preferably contained in the second reagent.

A combination of peroxidase and one of the two oxidative coupling-type chromogens used for oxidative coupling coloring reaction which is used for the elimination of hydrogen peroxide formed in the first reaction is preferably contained in the first reagent.

A polyanion is preferably contained in the first reagent.

A divalent metal ion is preferably contained in the first reagent.

An anti-apoB antibody may be contained in both of the first reagent and the second reagent, but is preferably contained in the first reagent.

An anti-apoE antibody may be contained in both of the first reagent and the second reagent, but is preferably contained in the first reagent.

Surfactant B and a reagent for the measurement of hydrogen peroxide are preferably contained in the second reagent.

A phospholipid-hydrolyzing enzyme may be contained in either or both of the first reagent and the second reagent, but is preferably contained in the second reagent.

Certain embodiments of the kit for the measurement of RLP-C of the present invention are illustrated below, but they are not to be construed as limiting the scope of the present invention.

### Kit 1

### First reagent

Cholesterol oxidase, catalase and surfactant A

### Second reagent

Cholesterol oxidase, cholesterol esterase, a catalase inhibitor and surfactant B

### Kit 2

### First reagent

Cholesterol oxidase, cholesterol esterase, catalase and surfactant A

### Second reagent

Cholesterol oxidase, cholesterol esterase, a catalase inhibitor and surfactant B

### Kit 3

### First reagent

Cholesterol oxidase, catalase and surfactant A

### Second reagent

Cholesterol oxidase, cholesterol esterase, a catalase inhibitor, surfactant B and a reagent for the measurement of hydrogen peroxide

### Kit 4

### First reagent

Cholesterol oxidase, cholesterol esterase, catalase and surfactant A

### Second reagent

Cholesterol oxidase, cholesterol esterase, a catalase inhibitor, surfactant B and a reagent for the measurement of hydrogen peroxide

### Kit 5

### First reagent

Cholesterol dehydrogenase, reduced coenzyme oxidase, oxidized coenzyme, catalase and surfactant A

### Second reagent

Cholesterol esterase, cholesterol dehydrogenase, reduced coenzyme oxidase, oxidized coenzyme, a catalase inhibitor and surfactant B

### Kit 6

### First reagent

Cholesterol esterase, cholesterol dehydrogenase, reduced coenzyme oxidase, oxidized coenzyme, catalase and surfactant A

### Second reagent

Cholesterol esterase, cholesterol dehydrogenase, reduced coenzyme oxidase, oxidized coenzyme, a catalase inhibitor and surfactant B

### Kit 7

### First reagent

Cholesterol dehydrogenase, reduced coenzyme oxidase, oxidized coenzyme, catalase and surfactant A

### Second reagent

Cholesterol esterase, cholesterol dehydrogenase, reduced coenzyme oxidase, oxidized coenzyme, a catalase inhibitor, surfactant B and a reagent for the measurement of hydrogen peroxide

### Kit 8

### First reagent

Cholesterol esterase, cholesterol dehydrogenase, reduced coenzyme oxidase, oxidized coenzyme, catalase and surfactant A

### Second reagent

Cholesterol esterase, cholesterol dehydrogenase, reduced coenzyme oxidase, oxidized coenzyme, a catalase inhibitor, surfactant B and a reagent for the measurement of hydrogen peroxide

### Kit 9

### First reagent

Cholesterol oxidase, cholesterol dehydrogenase, reduced coenzyme oxidase, oxidized coenzyme, catalase and surfactant A

### Second reagent

Cholesterol esterase, cholesterol oxidase, cholesterol dehydrogenase, reduced coenzyme oxidase, oxidized coenzyme, a catalase inhibitor and surfactant B

### Kit 10

### First reagent

Cholesterol esterase, cholesterol oxidase, cholesterol dehydrogenase, reduced coenzyme oxidase, oxidized coenzyme, catalase and surfactant A

### Second reagent

Cholesterol esterase, cholesterol oxidase, cholesterol dehydrogenase, reduced coenzyme oxidase, oxidized coenzyme, a catalase inhibitor and surfactant B

### Kit 11

### First reagent

Cholesterol oxidase, cholesterol dehydrogenase, reduced coenzyme oxidase, oxidized coenzyme, catalase and surfactant A

### Second reagent

Cholesterol esterase, cholesterol oxidase, cholesterol dehydrogenase, reduced coenzyme oxidase, oxidized coenzyme, a catalase inhibitor, surfactant B and a reagent for the measurement of hydrogen peroxide

### Kit 12

### First reagent

Cholesterol esterase, cholesterol oxidase, cholesterol dehydrogenase, reduced coenzyme oxidase, oxidized coenzyme, catalase and surfactant A

### Second reagent

Cholesterol esterase, cholesterol oxidase, cholesterol dehydrogenase, reduced coenzyme oxidase, oxidized coenzyme, a catalase inhibitor, surfactant B and a reagent for the measurement of hydrogen peroxide

### Kit 13

### First reagent

Cholesterol oxidase, peroxidase, one of the two oxidative coupling-type chromogens and surfactant A

### Second reagent

Cholesterol esterase, cholesterol oxidase, surfactant B and the other oxidative coupling-type chromogen

### Kit 14

### First reagent

Cholesterol esterase, cholesterol oxidase, peroxidase, one of the two oxidative coupling-type chromogens and surfactant A

### Second reagent

Cholesterol esterase, cholesterol oxidase, surfactant B and the other oxidative coupling-type chromogen

### Kit 15

### First reagent

Cholesterol dehydrogenase, reduced coenzyme oxidase, oxidized coenzyme, peroxidase, one of the two oxidative coupling-type chromogens and surfactant A

### Second reagent

Cholesterol esterase, cholesterol dehydrogenase, reduced coenzyme oxidase, oxidized coenzyme, surfactant B and the other oxidative coupling-type chromogen

### Kit 16

### First reagent

Cholesterol esterase, cholesterol dehydrogenase, reduced coenzyme oxidase, oxidized coenzyme, peroxidase, one of the two oxidative coupling-type chromogens and surfactant A

### Second reagent

Cholesterol esterase, cholesterol dehydrogenase, reduced coenzyme oxidase, oxidized coenzyme, surfactant B and the other oxidative coupling-type chromogen

### Kit 17

### First reagent

Cholesterol dehydrogenase, reduced coenzyme oxidase, oxidized coenzyme, cholesterol oxidase, peroxidase, one of the two oxidative coupling-type chromogens and surfactant A

### Second reagent

Cholesterol esterase, cholesterol dehydrogenase, reduced coenzyme oxidase, oxidized coenzyme, cholesterol oxidase, surfactant B and the other oxidative coupling-type chromogen

### Kit 18

### First reagent

Cholesterol esterase, cholesterol dehydrogenase, reduced coenzyme oxidase, oxidized coenzyme, cholesterol oxidase, peroxidase, one of the two oxidative coupling-type chromogens and surfactant A

### Second reagent

Cholesterol esterase, cholesterol dehydrogenase, reduced coenzyme oxidase, oxidized coenzyme, cholesterol oxidase, surfactant B and the other oxidative coupling-type chromogen

### Kit 19

### First reagent

Cholesterol oxidase, a polyanion, catalase and surfactant (A1)

### Second reagent

Cholesterol esterase, cholesterol oxidase, a catalase inhibitor and surfactant B

### Kit 20

### First reagent

Cholesterol esterase, cholesterol oxidase, a polyanion, catalase and surfactant (A1)

### Second reagent

Cholesterol esterase, cholesterol oxidase, a catalase inhibitor and surfactant B

### Kit 21

### First reagent

Cholesterol oxidase, a polyanion, catalase and surfactant (A1)

### Second reagent

Cholesterol esterase, cholesterol oxidase, a catalase inhibitor, surfactant B and a reagent for the measurement of hydrogen peroxide

### Kit 22

### First reagent

Cholesterol esterase, cholesterol oxidase, a polyanion, catalase and surfactant (A1)

### Second reagent

Cholesterol esterase, cholesterol oxidase, a catalase inhibitor, surfactant B and a reagent for the measurement of hydrogen peroxide

### Kit 23

### First reagent

Cholesterol dehydrogenase, reduced coenzyme oxidase, oxidized coenzyme, a polyanion, catalase and surfactant (A1)

### Second reagent

Cholesterol esterase, cholesterol dehydrogenase, reduced coenzyme oxidase, oxidized coenzyme, a catalase inhibitor and surfactant B

### Kit 24

### First reagent

Cholesterol esterase, cholesterol dehydrogenase, reduced coenzyme oxidase, oxidized coenzyme, a polyanion, catalase and surfactant (A1)

### Second reagent

Cholesterol esterase, cholesterol dehydrogenase, reduced coenzyme oxidase, oxidized coenzyme, a catalase inhibitor and surfactant B

### Kit 25

### First reagent

Cholesterol dehydrogenase, reduced coenzyme oxidase, oxidized coenzyme, a polyanion, catalase and surfactant (A1)

### Second reagent

Cholesterol esterase, cholesterol dehydrogenase, reduced coenzyme oxidase, oxidized coenzyme, a catalase inhibitor, surfactant B and a reagent for the measurement of hydrogen peroxide

### Kit 26

### First reagent

Cholesterol esterase, cholesterol dehydrogenase, reduced coenzyme oxidase, oxidized coenzyme, a polyanion, catalase and surfactant (A1)

### Second reagent

Cholesterol esterase, cholesterol dehydrogenase, reduced coenzyme oxidase, oxidized coenzyme, a catalase inhibitor, surfactant B and a reagent for the measurement of hydrogen peroxide

### Kit 27

### First reagent

Cholesterol oxidase, cholesterol dehydrogenase, reduced coenzyme oxidase, oxidized coenzyme, a polyanion, catalase and surfactant (A1)

### Second reagent

Cholesterol esterase, cholesterol oxidase, cholesterol dehydrogenase, reduced coenzyme oxidase, oxidized coenzyme, a catalase inhibitor and surfactant B

### Kit 28

### First reagent

Cholesterol esterase, cholesterol oxidase, cholesterol dehydrogenase, reduced coenzyme oxidase, oxidized coenzyme, a polyanion, catalase and surfactant (A1)

### Second reagent

Cholesterol esterase, cholesterol oxidase, cholesterol dehydrogenase, reduced coenzyme oxidase, oxidized coenzyme, a catalase inhibitor and surfactant B

### Kit 29

### First reagent

Cholesterol oxidase, cholesterol dehydrogenase, reduced coenzyme oxidase, oxidized coenzyme, a polyanion, catalase and surfactant (A1)

### Second reagent

Cholesterol esterase, cholesterol oxidase, cholesterol dehydrogenase, reduced coenzyme oxidase, oxidized coenzyme, a catalase inhibitor, surfactant B and a reagent for the measurement of hydrogen peroxide

### Kit 30

### First reagent

Cholesterol esterase, cholesterol oxidase, cholesterol dehydrogenase, reduced coenzyme oxidase, oxidized coenzyme, a polyanion, catalase and surfactant (A1)

### Second reagent

Cholesterol esterase, cholesterol oxidase, cholesterol dehydrogenase, reduced coenzyme, oxidase, oxidized coenzyme, a catalase inhibitor, surfactant B and a reagent for the measurement of hydrogen peroxide

### Kit 31

### First reagent

Cholesterol oxidase, a polyanion, peroxidase, one of the two oxidative coupling-type chromogens and surfactant (A1)

### Second reagent

Cholesterol esterase, cholesterol oxidase, surfactant B and the other oxidative coupling-type chromogen

### Kit 32

### First reagent

Cholesterol esterase, cholesterol oxidase, a polyanion, peroxidase, one of the two oxidative coupling-type chromogens and surfactant (A1)

### Second reagent

Cholesterol esterase, cholesterol oxidase, surfactant B and the other oxidative coupling-type chromogen

### Kit 33

### First reagent

Cholesterol dehydrogenase, reduced coenzyme oxidase, oxidized coenzyme, a polyanion, peroxidase, one of the two oxidative coupling-type chromogens and surfactant (A1)

### Second reagent

Cholesterol esterase, cholesterol dehydrogenase, reduced coenzyme oxidase, oxidized coenzyme, surfactant B and the other oxidative coupling-type chromogen

### Kit 34

### First reagent

Cholesterol esterase, cholesterol dehydrogenase, reduced coenzyme oxidase, oxidized coenzyme, a polyanion, peroxidase, one of the two oxidative coupling-type chromogens and surfactant (A1)

### Second reagent

Cholesterol esterase, cholesterol dehydrogenase, reduced coenzyme oxidase, oxidized coenzyme, surfactant B and the other oxidative coupling-type chromogen

### Kit 35

### First reagent

Cholesterol oxidase, cholesterol dehydrogenase, reduced coenzyme oxidase, oxidized coenzyme, a polyanion, peroxidase, one of the two oxidative coupling-type chromogens and surfactant (A1)

### Second reagent

Cholesterol esterase, cholesterol oxidase, cholesterol dehydrogenase, reduced coenzyme oxidase, oxidized coenzyme, surfactant B and the other oxidative coupling-type chromogen

### Kit 36

### First reagent

Cholesterol esterase, cholesterol oxidase, cholesterol dehydrogenase, reduced coenzyme oxidase, oxidized coenzyme, a polyanion, peroxidase, one of the two oxidative coupling-type chromogens and surfactant (A1)

### Second reagent

Cholesterol esterase, cholesterol oxidase, cholesterol dehydrogenase, reduced coenzyme oxidase, oxidized coenzyme, surfactant B and the other oxidative coupling-type chromogen

### Kit 37

### First reagent

Cholesterol oxidase, a polyanion, a divalent metal ion, catalase and surfactant (A2)

### Second reagent

Cholesterol esterase, cholesterol oxidase, a catalase inhibitor and surfactant B

### Kit 38

### First reagent

Cholesterol esterase, cholesterol oxidase, a polyanion, a divalent metal ion, catalase and surfactant (A2)

### Second reagent

Cholesterol esterase, cholesterol oxidase, a catalase inhibitor and surfactant B

### Kit 39

### First reagent

Cholesterol oxidase, a polyanion, a divalent metal ion, catalase and surfactant (A2)

### Second reagent

Cholesterol esterase, cholesterol oxidase, a catalase inhibitor, surfactant B and a reagent for the measurement of hydrogen peroxide

### Kit 40

### First reagent

Cholesterol esterase, cholesterol oxidase, a polyanion, a divalent metal ion, catalase and surfactant (A2)

### Second reagent

Cholesterol esterase, cholesterol oxidase, a catalase inhibitor, surfactant B and a reagent for the measurement of hydrogen peroxide

### Kit 41

### First reagent

Cholesterol oxidase, an anti-apoB antibody and/or an anti-apoE antibody, catalase and surfactant (A2)

### Second reagent

Cholesterol esterase, cholesterol oxidase, a catalase inhibitor and surfactant B

### Kit 42

### First reagent

Cholesterol esterase, cholesterol oxidase, an anti-apoB antibody and/or an anti-apoE antibody, catalase and surfactant (A2)

### Second reagent

Cholesterol esterase, cholesterol oxidase, a catalase inhibitor and surfactant B

### Kit 43

### First reagent

Cholesterol oxidase, an anti-apoB antibody and/or an anti-apoE antibody, catalase and surfactant (A2)

### Second reagent

Cholesterol esterase, cholesterol oxidase, a catalase inhibitor, surfactant B and a reagent for the measurement of hydrogen peroxide

### Kit 44

### First reagent

Cholesterol esterase, cholesterol oxidase, an anti-apoB antibody and/or an anti-apoE antibody, catalase and surfactant (A2)

### Second reagent

Cholesterol esterase, cholesterol oxidase, a catalase inhibitor, surfactant B and a reagent for the measurement of hydrogen peroxide

### Kit 45

### First reagent

Cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol dehydrogenase, reduced coenzyme oxidase, oxidized coenzyme, a catalase inhibitor and surfactant B

### Kit 46

### First reagent

Cholesterol esterase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol dehydrogenase, reduced coenzyme oxidase, oxidized coenzyme, a catalase inhibitor and surfactant B

### Kit 47

### First reagent

Cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol dehydrogenase, reduced coenzyme oxidase, oxidized coenzyme, a catalase inhibitor, surfactant B and a reagent for the measurement of hydrogen peroxide

### Kit 48

### First reagent

Cholesterol esterase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol dehydrogenase, reduced coenzyme oxidase, oxidized coenzyme, a catalase inhibitor, surfactant B and a reagent for the measurement of hydrogen peroxide

### Kit 49

### First reagent

Cholesterol oxidase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol oxidase, cholesterol dehydrogenase, reduced coenzyme oxidase, oxidized coenzyme, a catalase inhibitor and surfactant B

### Kit 50

### First reagent

Cholesterol esterase, cholesterol oxidase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol oxidase, cholesterol dehydrogenase, reduced coenzyme oxidase, oxidized coenzyme, a catalase inhibitor and surfactant B

### Kit 51

### First reagent

Cholesterol oxidase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol oxidase, cholesterol dehydrogenase, reduced coenzyme oxidase, oxidized coenzyme, a catalase inhibitor, surfactant B and a reagent for the measurement of hydrogen peroxide

### Kit 52

### First reagent

Cholesterol esterase, cholesterol oxidase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol oxidase, cholesterol dehydrogenase, reduced coenzyme oxidase, oxidized coenzyme, a catalase inhibitor, surfactant B and a reagent for the measurement of hydrogen peroxide

### Kit 53

### First reagent

Cholesterol oxidase, a polyanion, a divalent metal ion, peroxidase, one of the two oxidative coupling-type chromogens and surfactant (A2)

### Second reagent

Cholesterol esterase, cholesterol oxidase, surfactant B and the other oxidative coupling-type chromogen

### Kit 54

### First reagent

Cholesterol esterase, cholesterol oxidase, a polyanion, a divalent metal ion, peroxidase, one of the two oxidative coupling-type chromogens and surfactant (A2)

### Second reagent

Cholesterol esterase, cholesterol oxidase, surfactant B and the other oxidative coupling-type chromogen

### Kit 55

### First reagent

Cholesterol dehydrogenase, reduced coenzyme oxidase, oxidized coenzyme, a polyanion, a divalent metal ion, peroxidase, one of the two oxidative coupling-type chromogens and surfactant (A2)

### Second reagent

Cholesterol esterase, cholesterol dehydrogenase, reduced coenzyme oxidase, oxidized coenzyme, surfactant B and the other oxidative coupling-type chromogen

### Kit 56

### First reagent

Cholesterol esterase, cholesterol dehydrogenase, reduced coenzyme oxidase, oxidized coenzyme, a polyanion, a divalent metal ion, peroxidase, one of the two oxidative coupling-type chromogens and surfactant (A2)

### Second reagent

Cholesterol esterase, cholesterol dehydrogenase, reduced coenzyme oxidase, oxidized coenzyme, surfactant B and the other oxidative coupling-type chromogen

### Kit 57

### First reagent

Cholesterol oxidase, cholesterol dehydrogenase, reduced coenzyme oxidase, oxidized coenzyme, a polyanion, a divalent metal ion, peroxidase, one of the two oxidative coupling-type chromogens and surfactant (A2)

### Second reagent

Cholesterol esterase, cholesterol oxidase, cholesterol dehydrogenase, reduced coenzyme oxidase, oxidized coenzyme, surfactant B and the other oxidative coupling-type chromogen

### Kit 58

### First reagent

Cholesterol esterase, cholesterol oxidase, cholesterol dehydrogenase, reduced coenzyme oxidase, oxidized coenzyme, a polyanion, a divalent metal ion, peroxidase, one of the two oxidative coupling-type chromogens and surfactant (A2)

### Second reagent

Cholesterol esterase, cholesterol oxidase, cholesterol dehydrogenase, reduced coenzyme oxidase, oxidized coenzyme, surfactant B and the other oxidative coupling-type chromogen

### Kit 59

### First reagent

Cholesterol oxidase, an anti-apoB antibody and/or an anti-apoE antibody, peroxidase, one of the two oxidative coupling-type chromogens and surfactant (A2)

### Second reagent

Cholesterol esterase, cholesterol oxidase, surfactant B and the other oxidative coupling-type chromogen

### Kit 60

### First reagent

Cholesterol esterase, cholesterol oxidase, an anti-apoB antibody and/or an anti-apoE antibody, peroxidase, one of the two oxidative coupling-type chromogens and surfactant (A2)

### Second reagent

Cholesterol esterase, cholesterol oxidase, surfactant B and the other oxidative coupling-type chromogen

### Kit 61

### First reagent

Cholesterol dehydrogenase, reduced coenzyme oxidase, oxidized coenzyme, an anti-apoB antibody and/or an anti-apoE antibody, peroxidase, one of the two oxidative coupling-type chromogens and surfactant (A2)

### Second reagent

Cholesterol esterase, cholesterol dehydrogenase, reduced coenzyme oxidase, oxidized coenzyme, surfactant B and the other oxidative coupling-type chromogen

### Kit 62

### First reagent

Cholesterol esterase, cholesterol dehydrogenase, reduced coenzyme oxidase, oxidized coenzyme, an anti-apoB antibody and/or an anti-apoE antibody, peroxidase, one of the two oxidative coupling-type chromogens and surfactant (A2)

### Second reagent

Cholesterol esterase, cholesterol dehydrogenase, reduced coenzyme oxidase, oxidized coenzyme, surfactant B and the other oxidative coupling-type chromogen

### Kit 63

### First reagent

Cholesterol oxidase, cholesterol dehydrogenase, reduced coenzyme oxidase, oxidized coenzyme, an anti-apoB antibody and/or an anti-apoE antibody, peroxidase, one of the two oxidative coupling-type chromogens and surfactant (A2)

### Second reagent

Cholesterol esterase, cholesterol oxidase, cholesterol dehydrogenase, reduced coenzyme oxidase, oxidized coenzyme, surfactant B and the other oxidative coupling-type chromogen

### Kit 64

### First reagent

Cholesterol esterase, cholesterol oxidase, cholesterol dehydrogenase, reduced coenzyme oxidase, oxidized coenzyme, an anti-apoB antibody and/or an anti-apoE antibody, peroxidase, one of the two oxidative coupling-type chromogens and surfactant (A2)

### Second reagent

Cholesterol esterase, cholesterol oxidase, cholesterol dehydrogenase, reduced coenzyme oxidase, oxidized coenzyme, surfactant B and the other oxidative coupling-type chromogen

### Kit 65

### First reagent

Cholesterol oxidase, catalase and surfactant A

### Second reagent

Cholesterol oxidase, cholesterol esterase, a phospholipid-hydrolyzing enzyme, a catalase inhibitor and surfactant B

### Kit 66

### First reagent

Cholesterol oxidase, cholesterol esterase, catalase and surfactant A

### Second reagent

Cholesterol oxidase, cholesterol esterase, a phospholipid-hydrolyzing enzyme, a catalase inhibitor and surfactant B

### Kit 67

### First reagent

Cholesterol oxidase, catalase and surfactant A

### Second reagent

Cholesterol oxidase, cholesterol esterase, a phospholipid-hydrolyzing enzyme, a catalase inhibitor, surfactant B and a reagent for the measurement of hydrogen peroxide

### Kit 68

### First reagent

Cholesterol oxidase, cholesterol esterase, catalase and surfactant A

### Second reagent

Cholesterol oxidase, cholesterol esterase, a phospholipid-hydrolyzing enzyme, a catalase inhibitor, surfactant B and a reagent for the measurement of hydrogen peroxide

### Kit 69

### First reagent

Cholesterol dehydrogenase, reduced coenzyme oxidase, oxidized coenzyme, catalase and surfactant A

### Second reagent

Cholesterol esterase, cholesterol dehydrogenase, reduced coenzyme oxidase, oxidized coenzyme, a phospholipid-hydrolyzing enzyme, a catalase inhibitor and surfactant B

### Kit 70

### First reagent

Cholesterol esterase, cholesterol dehydrogenase, reduced coenzyme oxidase, oxidized coenzyme, catalase and surfactant A

### Second reagent

Cholesterol esterase, cholesterol dehydrogenase, reduced coenzyme oxidase, oxidized coenzyme, a phospholipid-hydrolyzing enzyme, a catalase inhibitor and surfactant B

### Kit 71

### First reagent

Cholesterol dehydrogenase, reduced coenzyme oxidase, oxidized coenzyme, catalase and surfactant A

### Second reagent

Cholesterol esterase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 72

### First reagent

Cholesterol esterase, cholesterol dehydrogenase, reduced coenzyme oxidase, oxidized coenzyme, catalase and surfactant A

### Second reagent

Cholesterol esterase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 73

### First reagent

Cholesterol oxidase, cholesterol dehydrogenase, reduced coenzyme oxidase, oxidized coenzyme, catalase and surfactant A

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor and surfactant B

### Kit 74

### First reagent

Cholesterol esterase, cholesterol oxidase, cholesterol dehydrogenase, reduced coenzyme oxidase, oxidized coenzyme, catalase and surfactant A

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor and surfactant B

### Kit 75

### First reagent

Cholesterol oxidase, cholesterol dehydrogenase, reduced coenzyme oxidase, oxidized coenzyme, catalase and surfactant A

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 76

### First reagent

Cholesterol esterase, cholesterol oxidase, cholesterol dehydrogenase, reduced coenzyme oxidase, oxidized coenzyme, catalase and surfactant A

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 77

### First reagent

Cholesterol oxidase, peroxidase, one of the two oxidative coupling-type chromogens and surfactant A

### Second reagent

Cholesterol esterase, cholesterol oxidase, a phospholipid-hydrolyzing enzyme, surfactant B and the other oxidative coupling-type chromogen

### Kit 78

### First reagent

Cholesterol esterase, cholesterol oxidase, peroxidase, one of the two oxidative coupling-type chromogens and surfactant A

### Second reagent

Cholesterol esterase, cholesterol oxidase, a phospholipid-hydrolyzing enzyme, surfactant B and the other oxidative coupling-type chromogen

### Kit 79

### First reagent

Cholesterol dehydrogenase, reduced coenzyme oxidase, oxidized coenzyme, peroxidase, one of the two oxidative coupling-type chromogens and surfactant A

### Second reagent

Cholesterol esterase, cholesterol dehydrogenase, reduced coenzyme oxidase, oxidized coenzyme, a phospholipid-hydrolyzing enzyme, surfactant B and the other oxidative coupling-type chromogen

### Kit 80

### First reagent

Cholesterol esterase, cholesterol dehydrogenase, reduced coenzyme oxidase, oxidized coenzyme, peroxidase, one of the two oxidative coupling-type chromogens and surfactant A

### Second reagent

Cholesterol esterase, cholesterol dehydrogenase, reduced coenzyme oxidase, oxidized coenzyme, a phospholipid-hydrolyzing enzyme, surfactant B and the other oxidative coupling-type chromogen

### Kit 81

### First reagent

Cholesterol oxidase, cholesterol dehydrogenase, reduced coenzyme oxidase, oxidized coenzyme, peroxidase, one of the two oxidative coupling-type chromogens and surfactant A

### Second reagent

Cholesterol esterase, cholesterol oxidase, cholesterol dehydrogenase, reduced coenzyme oxidase, oxidized coenzyme, a phospholipid-hydrolyzing enzyme, surfactant B and the other oxidative coupling-type chromogen

### Kit 82

### First reagent

Cholesterol esterase, cholesterol dehydrogenase, reduced coenzyme oxidase, oxidized coenzyme, cholesterol oxidase, peroxidase, one of the two oxidative coupling-type chromogens and surfactant A

### Second reagent

Cholesterol esterase, cholesterol oxidase, cholesterol dehydrogenase, reduced coenzyme oxidase, oxidized coenzyme, a phospholipid-hydrolyzing enzyme, surfactant B and the other oxidative coupling-type chromogen

### Kit 83

### First reagent

Cholesterol oxidase, a polyanion, catalase and surfactant (A1)

### Second reagent

Cholesterol esterase, cholesterol oxidase, a phospholipid-hydrolyzing enzyme, a catalase inhibitor and surfactant B

### Kit 84

### First reagent

Cholesterol esterase, cholesterol oxidase, a polyanion, catalase and surfactant (A1)

### Second reagent

Cholesterol esterase, cholesterol oxidase, a phospholipid-hydrolyzing enzyme, a catalase inhibitor and surfactant B

### Kit 85

### First reagent

Cholesterol oxidase, a polyanion, catalase and surfactant (A1)

### Second reagent

Cholesterol esterase, cholesterol oxidase, a phospholipid-hydrolyzing enzyme, a catalase inhibitor, surfactant B and a reagent for the measurement of hydrogen peroxide

### Kit 86

### First reagent

Cholesterol esterase, cholesterol oxidase, a polyanion, catalase and surfactant (A1)

### Second reagent

Cholesterol esterase, cholesterol oxidase, a phospholipid-hydrolyzing enzyme, a catalase inhibitor, surfactant B and a reagent for the measurement of hydrogen peroxide

### Kit 87

### First reagent

Cholesterol dehydrogenase, reduced coenzyme oxidase, oxidized coenzyme, a polyanion, catalase and surfactant (A1)

### Second reagent

Cholesterol esterase, cholesterol dehydrogenase, reduced coenzyme oxidase, oxidized coenzyme, a phospholipid-hydrolyzing enzyme, a catalase inhibitor and surfactant B

### Kit 88

### First reagent

Cholesterol esterase, cholesterol dehydrogenase, reduced coenzyme oxidase, oxidized coenzyme, a polyanion, catalase and surfactant (A1)

### Second reagent

Cholesterol esterase, cholesterol dehydrogenase, reduced coenzyme oxidase, oxidized coenzyme, a phospholipid-hydrolyzing enzyme, a catalase inhibitor and surfactant B

### Kit 89

### First reagent

Cholesterol dehydrogenase, reduced coenzyme oxidase, oxidized coenzyme, a polyanion, catalase and surfactant (A1)

### Second reagent

Cholesterol esterase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 90

### First reagent

Cholesterol esterase, cholesterol dehydrogenase, reduced coenzyme oxidase, oxidized coenzyme, a polyanion, catalase and surfactant (A1)

### Second reagent

Cholesterol esterase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 91

### First reagent

Cholesterol oxidase, cholesterol dehydrogenase, reduced coenzyme oxidase, oxidized coenzyme, a polyanion, catalase and surfactant (A1)

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor and surfactant B

### Kit 92

### First reagent

Cholesterol esterase, cholesterol oxidase, cholesterol dehydrogenase, reduced coenzyme oxidase, oxidized coenzyme, a polyanion, catalase and surfactant (A1)

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor and surfactant B

### Kit 93

### First reagent

Cholesterol oxidase, cholesterol dehydrogenase, reduced coenzyme oxidase, oxidized coenzyme, a polyanion, catalase and surfactant (A1)

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 94

### First reagent

Cholesterol esterase, cholesterol oxidase, cholesterol dehydrogenase, reduced coenzyme oxidase, oxidized coenzyme, a polyanion, catalase and surfactant (A1)

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 95

### First reagent

Cholesterol oxidase, a polyanion, peroxidase, one of the two oxidative coupling-type chromogens and surfactant (A1)

### Second reagent

Cholesterol esterase, cholesterol oxidase, a phospholipid-hydrolyzing enzyme, surfactant B and the other oxidative coupling-type chromogen

### Kit 96

### First reagent

Cholesterol esterase, cholesterol oxidase, a polyanion, peroxidase, one of the two oxidative coupling-type chromogens and surfactant (A1)

### Second reagent

Cholesterol esterase, cholesterol oxidase, a phospholipid-hydrolyzing enzyme, surfactant B and the other oxidative coupling-type chromogen

### Kit 97

### First reagent

Cholesterol dehydrogenase, reduced coenzyme oxidase, oxidized coenzyme, a polyanion, peroxidase, one of the two oxidative coupling-type chromogens and surfactant (A1)

### Second reagent

Cholesterol esterase, cholesterol dehydrogenase, reduced coenzyme oxidase, oxidized coenzyme, a phospholipid-hydrolyzing enzyme, surfactant B and the other oxidative coupling-type chromogen

### Kit 98

### First reagent

Cholesterol esterase, cholesterol dehydrogenase, reduced coenzyme oxidase, oxidized coenzyme, a polyanion, peroxidase, one of the two oxidative coupling-type chromogens and surfactant (A1)

### Second reagent

Cholesterol esterase, cholesterol dehydrogenase, reduced coenzyme oxidase, oxidized coenzyme, a phospholipid-hydrolyzing enzyme, surfactant B and the other oxidative coupling-type chromogen

### Kit 99

### First reagent

Cholesterol oxidase, cholesterol dehydrogenase, reduced coenzyme oxidase, oxidized coenzyme, a polyanion, peroxidase, one of the two oxidative coupling-type chromogens and surfactant (A1)

### Second reagent

Cholesterol esterase, cholesterol oxidase, cholesterol dehydrogenase, reduced coenzyme oxidase, oxidized coenzyme, a phospholipid-hydrolyzing enzyme, surfactant B and the other oxidative coupling-type chromogen

### Kit 100

### First reagent

Cholesterol esterase, cholesterol oxidase, cholesterol dehydrogenase, reduced coenzyme oxidase, oxidized coenzyme, a polyanion, peroxidase, one of the two oxidative coupling-type chromogens and surfactant (A1)

### Second reagent

Cholesterol esterase, cholesterol oxidase, cholesterol dehydrogenase, reduced coenzyme oxidase, oxidized coenzyme, a phospholipid-hydrolyzing enzyme, surfactant B and the other oxidative coupling-type chromogen

### Kit 101

### First reagent

Cholesterol oxidase, a polyanion, a divalent metal ion, catalase and surfactant (A2)

### Second reagent

Cholesterol esterase, cholesterol oxidase, a phospholipid-hydrolyzing enzyme, a catalase inhibitor and surfactant B

### Kit 102

### First reagent

Cholesterol esterase, cholesterol oxidase, a polyanion, a divalent metal ion, catalase and surfactant (A2)

### Second reagent

Cholesterol esterase, cholesterol oxidase, a phospholipid-hydrolyzing enzyme, a catalase inhibitor and surfactant B

### Kit 103

### First reagent

Cholesterol oxidase, a polyanion, a divalent metal ion, catalase and surfactant (A2)

### Second reagent

Cholesterol esterase, cholesterol oxidase, a phospholipid-hydrolyzing enzyme, a catalase inhibitor, surfactant B and a reagent for the measurement of hydrogen peroxide

### Kit 104

### First reagent

Cholesterol esterase, cholesterol oxidase, a polyanion, a divalent metal ion, catalase and surfactant (A2)

### Second reagent

Cholesterol esterase, cholesterol oxidase, a phospholipid-hydrolyzing enzyme, a catalase inhibitor, surfactant B and a reagent for the measurement of hydrogen peroxide

### Kit 105

### First reagent

Cholesterol oxidase, an anti-apoB antibody and/or an anti-apoE antibody, catalase and surfactant (A2)

### Second reagent

Cholesterol esterase, cholesterol oxidase, a phospholipid-hydrolyzing enzyme, a catalase inhibitor and surfactant B

### Kit 106

### First reagent

Cholesterol esterase, cholesterol oxidase, an anti-apoB antibody and/or an anti-apoE antibody, catalase and surfactant (A2)

### Second reagent

Cholesterol esterase, cholesterol oxidase, a phospholipid-hydrolyzing enzyme, a catalase inhibitor and surfactant B

### Kit 107

### First reagent

Cholesterol oxidase, an anti-apoB antibody and/or an anti-apoE antibody, catalase and surfactant (A2)

### Second reagent

Cholesterol esterase, cholesterol oxidase, a phospholipid-hydrolyzing enzyme, a catalase inhibitor, surfactant B and a reagent for the measurement of hydrogen peroxide

### Kit 108

### First reagent

Cholesterol esterase, cholesterol oxidase, an anti-apoB antibody and/or an anti-apoE antibody, catalase and surfactant (A2)

### Second reagent

Cholesterol esterase, cholesterol oxidase, a phospholipid-hydrolyzing enzyme, a catalase inhibitor, surfactant B and a reagent for the measurement of hydrogen peroxide

### Kit 109

### First reagent

Cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol dehydrogenase, reduced coenzyme oxidase, oxidized coenzyme, a phospholipid-hydrolyzing enzyme, a catalase inhibitor and surfactant B

### Kit 110

### First reagent

Cholesterol esterase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol dehydrogenase, reduced coenzyme oxidase, oxidized coenzyme, a phospholipid-hydrolyzing enzyme, a catalase inhibitor and surfactant B

### Kit 111

### First reagent

Cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 112

### First reagent

Cholesterol esterase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 113

### First reagent

Cholesterol oxidase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor and surfactant B

### Kit 114

### First reagent

Cholesterol esterase, cholesterol oxidase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor and surfactant B

### Kit 115

### First reagent

Cholesterol oxidase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 116

### First reagent

Cholesterol esterase, cholesterol oxidase, cholesterol dehydrogenase, reduced coenzyme oxidase, oxidized coenzyme, an anti-apoB antibody and/or an anti-apoE antibody, catalase and surfactant (A2)

### Second reagent

Cholesterol esterase, cholesterol oxidase, cholesterol dehydrogenase, reduced coenzyme oxidase, oxidized coenzyme, a phospholipid-hydrolyzing enzyme, a catalase inhibitor, surfactant B and a reagent for the measurement of hydrogen peroxide

### Kit 117

### First reagent

Cholesterol oxidase, a polyanion, a divalent metal ion, peroxidase, one of the two oxidative coupling-type chromogens and surfactant (A2)

### Second reagent

Cholesterol esterase, cholesterol oxidase, a phospholipid-hydrolyzing enzyme, surfactant B and the other oxidative coupling-type chromogen

### Kit 118

### First reagent

Cholesterol esterase, cholesterol oxidase, a polyanion, a divalent metal ion, peroxidase, one of the two oxidative coupling-type chromogens and surfactant (A2)

### Second reagent

Cholesterol esterase, cholesterol oxidase, a phospholipid-hydrolyzing enzyme, surfactant B and the other oxidative coupling-type chromogen

### Kit 119

### First reagent

Cholesterol dehydrogenase, reduced coenzyme oxidase, oxidized coenzyme, a polyanion, a divalent metal ion, peroxidase, one of the two oxidative coupling-type chromogens and surfactant (A2)

### Second reagent

Cholesterol esterase, cholesterol dehydrogenase, reduced coenzyme oxidase, oxidized coenzyme, a phospholipid-hydrolyzing enzyme, surfactant B and the other oxidative coupling-type chromogen

### Kit 120

### First reagent

Cholesterol esterase, cholesterol dehydrogenase, reduced coenzyme oxidase, oxidized coenzyme, a polyanion, a divalent metal ion, peroxidase, one of the two oxidative coupling-type chromogens and surfactant (A2)

### Second reagent

Cholesterol esterase, cholesterol dehydrogenase, reduced coenzyme oxidase, oxidized coenzyme, a phospholipid-hydrolyzing enzyme, surfactant B and the other oxidative coupling-type chromogen

### Kit 121

### First reagent

Cholesterol oxidase, cholesterol dehydrogenase, reduced coenzyme oxidase, oxidized coenzyme, a polyanion, a divalent metal ion, peroxidase, one of the two oxidative coupling-type chromogens and surfactant (A2)

### Second reagent

Cholesterol esterase, cholesterol oxidase, cholesterol dehydrogenase, reduced coenzyme oxidase, oxidized coenzyme, a phospholipid-hydrolyzing enzyme, surfactant B and the other oxidative coupling-type chromogen

### Kit 122

### First reagent

Cholesterol esterase, cholesterol oxidase, cholesterol dehydrogenase, reduced coenzyme oxidase, oxidized coenzyme, a polyanion, a divalent metal ion, peroxidase, one of the two oxidative coupling-type chromogens and surfactant (A2)

### Second reagent

Cholesterol esterase, cholesterol oxidase, cholesterol dehydrogenase, reduced coenzyme oxidase, oxidized coenzyme, a phospholipid-hydrolyzing enzyme, surfactant B and the other oxidative coupling-type chromogen

### Kit 123

### First reagent

Cholesterol oxidase, an anti-apoB antibody and/or an anti-apoE antibody, peroxidase, one of the two oxidative coupling-type chromogens and surfactant (A2)

### Second reagent

Cholesterol esterase, cholesterol oxidase, a phospholipid-hydrolyzing enzyme, surfactant B and the other oxidative coupling-type chromogen

### Kit 124

### First reagent

Cholesterol esterase, cholesterol oxidase, an anti-apoB antibody and/or an anti-apoE antibody, peroxidase, one of the two oxidative coupling-type chromogens and surfactant (A2)

### Second reagent

Cholesterol esterase, cholesterol oxidase, a phospholipid-hydrolyzing enzyme, surfactant B and the other oxidative coupling-type chromogen

### Kit 125

### First reagent

Cholesterol dehydrogenase, reduced coenzyme oxidase, oxidized coenzyme, an anti-apoB antibody and/or an anti-apoE antibody, peroxidase, one of the two oxidative coupling-type chromogens and surfactant (A2)

### Second reagent

Cholesterol esterase, cholesterol dehydrogenase, reduced coenzyme oxidase, oxidized coenzyme, a phospholipid-hydrolyzing enzyme, surfactant B and the other oxidative coupling-type chromogen

### Kit 126

### First reagent

Cholesterol esterase, cholesterol dehydrogenase, reduced coenzyme oxidase, oxidized coenzyme, an anti-apoB antibody and/or an anti-apoE antibody, peroxidase, one of the two oxidative coupling-type chromogens and surfactant (A2)

### Second reagent

Cholesterol esterase, cholesterol dehydrogenase, reduced coenzyme oxidase, oxidized coenzyme, a phospholipid-hydrolyzing enzyme, surfactant B and the other oxidative coupling-type chromogen

### Kit 127

### First reagent

Cholesterol oxidase, cholesterol dehydrogenase, reduced coenzyme oxidase, oxidized coenzyme, an anti-apoB antibody and/or an anti-apoE antibody, peroxidase, one of the two oxidative coupling-type chromogens and surfactant (A2)

### Second reagent

Cholesterol esterase, cholesterol oxidase, cholesterol dehydrogenase, reduced coenzyme oxidase, oxidized coenzyme, a phospholipid-hydrolyzing enzyme, surfactant B and the other oxidative coupling-type chromogen

### Kit 128

### First reagent

Cholesterol esterase, cholesterol oxidase, cholesterol dehydrogenase, reduced coenzyme oxidase, oxidized coenzyme, an anti-apoB antibody and/or an anti-apoE antibody, peroxidase, one of the two oxidative coupling-type chromogens and surfactant (A2)

### Second reagent

Cholesterol esterase, cholesterol oxidase, cholesterol dehydrogenase, reduced coenzyme oxidase, oxidized coenzyme, a phospholipid-hydrolyzing enzyme, surfactant B and the other oxidative coupling-type chromogen

In the reagent and the kit for the measurement of RLP-C of the present invention, the following components which are described in the above description of the method for the measurement of RLP-C of the present invention can be used: cholesterol oxidase, surfactant A, surfactant (A1), surfactant (A2), a polyanion, a divalent metal ion, an anti-apoB antibody, an anti-apoE antibody, catalase, peroxidase, oxidative coupling-type chromogens, cholesterol esterase, surfactant B, a reagent for the measurement of hydrogen peroxide and a phospholipid-hydrolyzing enzyme.

The reagent and the kit for the measurement of RLP-C of the present invention may comprise, according to need, an aqueous medium, a stabilizer, an antiseptic, an interference inhibitor, a reaction promoter, etc. Examples of the aqueous media are the above-mentioned aqueous media. Examples of the stabilizers are ethylenediaminetetraacetic acid (EDTA), sucrose and calcium chloride. Examples of the antiseptics are sodium azide, Bioace and antibiotics. An example of the interference inhibitor is ascorbate oxidase to inhibit the effect of ascorbic acid. Examples of the reaction promoters are enzymes such as colipase, and salts such as sodium sulfate and sodium chloride.

The reagent and the kit for the measurement of RLP-C of the present invention may be in freeze-dried form or in a state of being dissolved in an aqueous medium. When RLP-C in a sample is measured using the reagent in freeze-dried form, the reagent is used after being dissolved in an aqueous medium.

Cholesterol esterase and cholesterol oxidase are contained in the reagent and the kit for the measurement of RLP-C of the present invention in such amount that the concentration thereof in an aqueous medium becomes preferably 0.001 to 2000 U/mL, more preferably 0.005 to 1000 U/mL.

Cholesterol dehydrogenase and reduced coenzyme oxidase are contained in the reagent and the kit for the measurement of RLP-C of the present invention in such amount that the concentration thereof in an aqueous medium becomes preferably 0.001 to 2000 U/mL, more preferably 0.005 to 1000 U/mL.

Oxidized coenzyme is contained in the reagent and the kit for the measurement of RLP-C of the present invention in such amount that the concentration thereof in an aqueous medium becomes preferably 0.01 to 500 mmol/L, more preferably 0.1 to 100 mmol/L.

A phospholipid-hydrolyzing enzyme is contained in the reagent and the kit for the measurement of RLP-C of the present invention in such amount that the concentration thereof in an aqueous medium becomes preferably 0.01 to 1000 U/mL, more preferably 0.1 to 500 U/mL.

Surfactant A, surfactant (A1) and surfactant (A2) are contained in the reagent and the kit for the measurement of RLP-C of the present invention in such amount that the concentration thereof in an aqueous medium becomes preferably 0.0001 to 10%, more preferably 0.001 to 5%.

A polyanion is contained in the reagent and the kit for the measurement of RLP-C of the present invention in such amount that the concentration thereof in an aqueous medium becomes preferably 0.0001 to 30%, more preferably 0.001 to 3%.

A divalent metal ion is contained in the reagent and the kit for the measurement of RLP-C of the present invention in such amount that the concentration thereof in an aqueous medium becomes preferably 0.001 to 30%, more preferably 0.01 to 3%.

Surfactant B is contained in the reagent and the kit for the measurement of RLP-C of the present invention in such amount that the concentration thereof in an aqueous medium becomes preferably 0.001 to 40%, more preferably 0.01 to 20%.

Certain specific embodiments of the present invention are illustrated in the following examples, which are not to be construed as limiting the scope of the present invention. Reagents and enzymes used in the examples and reference examples are products from the following manufacturers.

MOPS (Dojindo Laboratories), EMSE (Daito Chemix Corporation), TOOS (Dojindo Laboratories), sodium sulfate (Kanto Chemical Co., Inc.), dextran sodium sulfate (Meito Sangyo Co., Ltd.), magnesium nitrate hexahydrate (Wako Pure Chemical Industries, Ltd.), 4-aminoantipyrine (Saikyo Kasei Co., Ltd.), peroxidase (Toyobo Co., Ltd.), CHO-CE (cholesterol oxidase; Kikkoman Corporation), LPL-312 (cholesterol esterase; Toyobo Co., Ltd.), chemically modified LPL (cholesterol esterase; prepared by Kyowa Medex Co., Ltd.), LPBP (cholesterol esterase; Asahi Kasei Corporation), CEN (cholesterol esterase; Asahi Kasei Corporation), COE-301 (cholesterol esterase; Toyobo Co., Ltd.), PLDP (phospholipid-hydrolyzing enzyme; Asahi Kasei Corporation), PLA2 (phospholipid-hydrolyzing enzyme; Asahi Kasei Corporation) and ascorbate oxidase (Asahi Kasei Corporation).

Emulgen B-66 (surfactant A: POE polycyclic phenyl ether with an HLB value of less than 16.0; Kao Corporation), Newkalgen GP-120 (surfactant A: POE·POA polycyclic phenyl ether; Takemoto Oil & Fat Co., Ltd.), Newcol 2608-F (surfactant A: POE·POA polycyclic phenyl ether; Nippon Nyukazai Co., Ltd.), Pluronic TR-704 (surfactant A: ethylenediamine-POE·POP copolymer; Adeka Corporation), ethylenediamine PO40EO40 (surfactant A: ethylenediamine-POE·POP copolymer; NOF Corporation), Plysurf A219B (surfactant A: POE alkyl ether phosphoate; Dai-ichi Kogyo Seiyaku Co., Ltd.), Monogen 170T (surfactant A: higher alcohol sulfuric acid ester salt; Dai-ichi Kogyo Seiyaku Co., Ltd.), Nikkol NP-10 (surfactant A: POE alkylphenyl ether; Nikko Chemicals Co., Ltd.), Hitenol N-07 (surfactant A: POE alkylphenyl ether sulfuric acid ester salt; Dai-ichi Kogyo Seiyaku Co., Ltd.), Noigen SD300 (surfactant A: POE short-chain branched alkyl ether; Dai-ichi Kogyo Seiyaku Co., Ltd.), Persoft EF (surfactant A: POE alkyl ether sulfuric acid ester salt; NOF Corporation), Persoft EP (surfactant A: POE alkyl ether sulfuric acid ester salt; NOF Corporation), Persoft EFT (surfactant A: POE alkyl ether sulfuric acid ester salt; NOF Corporation), Persoft EDO (surfactant A: POE alkyl ether sulfuric acid ester salt; NOF Corporation), Newcol 707-SF (surfactant A: POE polycyclic phenyl ether sulfuric acid ester salt; Nippon Nyukazai Co., Ltd.), Newcol 707-SFC (surfactant A: POE polycyclic phenyl ether sulfuric acid ester salt; Nippon Nyukazai Co., Ltd.), Newcol 723-SF (surfactant A: POE polycyclic phenyl ether sulfuric acid ester salt; Nippon Nyukazai Co., Ltd.), Pluronic L-34 (surfactant A: POE·POP copolymer having a POP molecular weight of less than 2,750; Adeka Corporation), Pluronic F-68 (surfactant A: POE·POP copolymer having a POP molecular weight of less than 2,750; Adeka Corporation), Epan 420 (surfactant A: POE·POP copolymer having a POP molecular weight of less than 2,750; Dai-ichi Kogyo Seiyaku Co., Ltd.), Epan U108 (surfactant A: POE·POP copolymer having a POP molecular weight of less than 2,750; Dai-ichi Kogyo Seiyaku Co., Ltd.), BLAUNON EP-1461 (surfactant A: POE·POP copolymer having a POP molecular weight of less than 2,750; Aoki Oil Industrial Co., Ltd.), Noigen XL-400 (POE·POA short-chain branched alkyl ether; Dai-ichi Kogyo Seiyaku Co., Ltd.), Noigen XL-1000F (POE·POA short-chain branched alkyl ether; Dai-ichi Kogyo Seiyaku Co., Ltd.), Unisafe PKA-5015 (surfactant A: POE·POA alkyl ether; NOF Corporation), Unisafe PKA-5016 (surfactant A: POE·POA alkyl ether; NOF Corporation), Unisafe LM-2 (surfactant A: POE·POA alkyl ether; NOF Corporation), EMALEX DAPE-230 (surfactant A: POE·POA alkyl ether; Nihon Emulsion Co., Ltd.), BLAUNON BEO-10 (surfactant A: POE bisphenol-A-ether; Aoki Oil Industrial Co., Ltd.), Newcol B4-SN (surfactant A: POE aryl ether sulfuric acid ester salt; Nippon Nyukazai Co., Ltd.), Newcol B13-SN (surfactant A: POE aryl ether sulfuric acid ester salt; Nippon Nyukazai Co., Ltd.), BIGCHAP (bile acid derivative; Dojindo Laboratories), deoxy-BIGCHAP (bile acid derivative; Dojindo Laboratories), CHAPSO (bile acid derivative; Dojindo Laboratories) and BLAUNON MXDA-PO4 (surfactant A: POP methaxylene diamine; Aoki Oil Industrial Co., Ltd.).

Ethylenediamine PO40EO40 [surfactant (A1): ethylenediamine-POE·POP copolymer; NOF Corporation], Finesurf IDEP-802 [surfactant (A1): POE·POA short-chain branched alkyl ether; Aoki Oil Industrial Co., Ltd.], Pluronic 25R-2 [surfactant (A1): POE·POP copolymer having a POP molecular weight of less than 2,750; Adeka Corporation), sodium cholate [surfactant (A1): bile acid derivative; Kanto Chemical Co., Inc.], Hitenol N-07 [surfactant (A1): POE alkylphenyl ether sulfuric acid ester salt; Dai-ichi Kogyo Seiyaku Co., Ltd.], Emulgen 810 [surfactant (a): POE alkylphenyl ether: Kao Corporation], Noigen SD110 [surfactant (A1): POE short-chain branched alkyl ether: Dai-ichi Kogyo Seiyaku Co., Ltd.], Noigen SD150 [surfactant (A1): POE short-chain branched alkyl ether: Dai-ichi Kogyo Seiyaku Co., Ltd.] and Noigen SD300 [surfactant (A1): POE short-chain branched alkyl ether: Dai-ichi Kogyo Seiyaku Co., Ltd.].

Newcol 707-F [surfactant (A2): POE·POA polycyclic phenyl ether; Nippon Nyukazai Co., Ltd.], Newcol 707-SN [surfactant (A2): POE polycyclic phenyl ether sulfuric acid ester salt; Nippon Nyukazai Co., Ltd.], EMALEX DAPE-230 [surfactant (A2): POE·POA alkyl ether; Nihon Emulsion Co., Ltd.], Newcol 1105-SN [surfactant (A2): POE alkyl ether sulfuric acid ester salt; Nippon Nyukazai Co., Ltd.], BLAUNON EP-1461 [surfactant (A2): POE·POP copolymer having a POP molecular weight of less than 2,750; Aoki Oil Industrial Co., Ltd.], sodium cholate [surfactant (A2): bile acid derivative; Kanto Chemical Co., Inc.], Emulgen 810 [surfactant (A2): POE alkylphenyl ether; Kao Corporation], Pluronic TR-704 [surfactant (A2): ethylenediamine-POE·POP copolymer; Adeka Corporation) and Finesurf IDEP-802 [surfactant (A2): POE·POA short-chain branched alkyl ether; Aoki Oil Industrial Co., Ltd.].

Pronon B-208 (surfactant B: POE·POB copolymer; NOF Corporation), BLAUNON TSP-50 [surfactant B: POE polycyclic phenyl ether with an HLB value of not less than 16.0; Aoki Oil Industrial Co., Ltd.], Emulgen A-500 [surfactant B: POE polycyclic phenyl ether with an HLB value of not less than 16.0; Kao Corporation], Newcol 740 [surfactant B: POE polycyclic phenyl ether with an HLB value of not less than 16.0; Nippon Nyukazai Co., Ltd.], Unilube MT-0620B (surfactant B: POE·POA long-chain branched alkyl ether; NOF Corporation), Pluronic F-108 [surfactant B: POE·POP copolymer having a POP molecular weight of not less than 2,750; Adeka Corporation), EMALEX 2425 (POE long-chain branched alkyl ether; Nippon Nyukazai Co., Ltd.), Noigen TDS-500F (POE long-chain branched alkyl ether; Dai-ichi Kogyo Seiyaku Co., Ltd.) and Wondersurf RL-100 (POE·POA alkyl ether; Aoki Oil Industrial Co., Ltd.).

### Example 1

A kit for the measurement of RLP-C comprising the following first reagent (Reagent A) and second reagent (Reagent a) was prepared.

| First reagent (Reagent A) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| Sodium cholate | 0.75 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 2

A kit for the measurement of RLP-C comprising the following first reagent (Reagent B) and second reagent (Reagent a) was prepared.

| First reagent (Reagent B) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| Emulgen B-66 | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 3

A kit for the measurement of RLP-C comprising the following first reagent (Reagent C) and second reagent (Reagent a) was prepared.

| First reagent (Reagent C) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| Pluronic TR-704 | 5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 4

A kit for the measurement of RLP-C comprising the following first reagent (Reagent D) and second reagent (Reagent a) was prepared.

| First reagent (Reagent D) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| Newcol 1105-SN | 2 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 5

A kit for the measurement of RLP-C comprising the following first reagent (Reagent E) and second reagent (Reagent a) was prepared.

| First reagent (Reagent E) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 2 kU/L |
| Emulgen B-66 | 2.5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronoun B-208 | 20 g/L |

### Example 6

A kit for the measurement of RLP-C comprising the following first reagent (Reagent F) and second reagent (Reagent a) was prepared.

| First reagent (Reagent F) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 1 kU/L |
| Newkalgen GP-120 | 4 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 7

A kit for the measurement of RLP-C comprising the following first reagent (Reagent G) and second reagent (Reagent a) was prepared.

| First reagent (Reagent G) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 1 kU/L |
| Newcol 2608-F | 4 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 8

A kit for the measurement of RLP-C comprising the following first reagent (Reagent H) and second reagent (Reagent a) was prepared.

| First reagent (Reagent H) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 1 kU/L |
| Pluronic TR-704 | 5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 9

A kit for the measurement of RLP-C comprising the following first reagent (Reagent I) and second reagent (Reagent a) was prepared.

| First reagent (Reagent I) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 1 kU/L |
| Plysurf A219B | 0.5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 10

A kit for the measurement of RLP-C comprising the following first reagent (Reagent J) and second reagent (Reagent a) was prepared.

| First reagent (Reagent J) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 1 kU/L |
| Monogen 170T | 3 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 11

A kit for the measurement of RLP-C comprising the following first reagent (Reagent K) and second reagent (Reagent a) was prepared.

| First reagent (Reagent K) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| LPBP | 600 kU/L |
| Nikkol NP-10 | 0.1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 12

A kit for the measurement of RLP-C comprising the following first reagent (Reagent L) and second reagent (Reagent a) was prepared.

| First reagent (Reagent L) | |
|---|---|
| MOPS (pH 6.5) | 4 g/L |
| EMSE | 0.3 g/L |
| Peroxidase | 10 kU/L |
| CHO-CE | 2 kU/L |
| Chemically modified LPL | 0.05 kU/L |
| Hitenol N-07 | 2 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 13

A kit for the measurement of RLP-C comprising the following first reagent (Reagent M) and second reagent (Reagent a) was prepared.

| First reagent (Reagent M) | |
|---|---|
| MOPS (pH 6.5) | 4 g/L |
| EMSE | 0.3 g/L |
| Peroxidase | 10 kU/L |
| CHO-CE | 2 kU/L |
| Chemically modified LPL | 0.05 kU/L |
| Noigen SD300 | 3 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 14

A kit for the measurement of RLP-C comprising the following first reagent (Reagent N) and second reagent (Reagent a) was prepared.

| First reagent (Reagent N) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| Ethylenediamine PO40EO40 | 0.25 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 15

A kit for the measurement of RLP-C comprising the following first reagent (Reagent O) and second reagent (Reagent a) was prepared.

| First reagent (Reagent O) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| Finesurf IDEP-802 | 0.1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 16

A kit for the measurement of RLP-C comprising the following first reagent (Reagent P) and second reagent (Reagent a) was prepared.

| First reagent (Reagent P) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| Pluronic 25R-2 | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 17

A kit for the measurement of RLP-C comprising the following first reagent (Reagent Q) and second reagent (Reagent a) was prepared.

| First reagent (Reagent Q) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| Hitenol N-07 | 2 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 18

A kit for the measurement of RLP-C comprising the following first reagent (Reagent R) and second reagent (Reagent a) was prepared.

| First reagent (Reagent R) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| Noigen SD110 | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 19

A kit for the measurement of RLP-C comprising the following first reagent (Reagent S) and second reagent (Reagent a) was prepared.

| First reagent (Reagent S) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| Noigen SD150 | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 20

A kit for the measurement of RLP-C comprising the following first reagent (Reagent T) and second reagent (Reagent a) was prepared.

| First reagent (Reagent T) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| Noigen SD300 | 3 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 21

A kit for the measurement of RLP-C comprising the following first reagent (Reagent U) and second reagent (Reagent a) was prepared.

| First reagent (Reagent U) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| Emulgen 810 | 0.05 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 22

A kit for the measurement of RLP-C comprising the following first reagent (Reagent V) and second reagent (Reagent a) was prepared.

| First reagent (Reagent V) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| COE-301 | 1 kU/L |
| Newcol 707-F | 2 g/L |
| | |

| Second reagent (Reagent a) | |
|---|---|
| | |
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 23

A kit for the measurement of RLP-C comprising the following first reagent (Reagent W) and second reagent (Reagent a) was prepared.

| First reagent (Reagent W) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| COE-301 | 1 kU/L |
| Newcol 707-SN | 4 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 24

A kit for the measurement of RLP-C comprising the following first reagent (Reagent X) and second reagent (Reagent a) was prepared.

| First reagent (Reagent X) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| COE-301 | 1 kU/L |
| EMALEX DAPE-230 | 2 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 25

A kit for the measurement of RLP-C comprising the following first reagent (Reagent Y) and second reagent (Reagent a) was prepared.

| First reagent (Reagent Y) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| COE-301 | 1 kU/L |
| Newcol 1105-SN | 2 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 26

A kit for the measurement of RLP-C comprising the following first reagent (Reagent Z) and second reagent (Reagent a) was prepared.

| First reagent (Reagent Z) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| Chemically modified LPL | 0.2 kU/L |
| Pluronic TR-704 | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 27

A kit for the measurement of RLP-C comprising the following first reagent (Reagent AA) and second reagent (Reagent a) was prepared.

| First reagent (Reagent AA) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| Chemically modified LPL | 0.2 kU/L |
| Finesurf IDEP-802 | 0.1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 28

A kit for the measurement of RLP-C comprising the following first reagent (Reagent AB) and second reagent (Reagent a) was prepared.

| First reagent (Reagent AB) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| Chemically modified LPL | 0.2 kU/L |
| BLAUNON EP-1461 | 4 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 29

A kit for the measurement of RLP-C comprising the following first reagent (Reagent AC) and second reagent (Reagent a) was prepared.

| First reagent (Reagent AC) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| Chemically modified LPL | 0.2 kU/L |
| Sodium cholate | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 30

A kit for the measurement of RLP-C comprising the following first reagent (Reagent AD) and second reagent (Reagent a) was prepared.

| First reagent (Reagent AD) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| LPBP | 600 kU/L |
| Emulgen 810 | 0.75 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 31

A kit for the measurement of RLP-C comprising the following first reagent (Reagent E) and second reagent (Reagent b) was prepared.

| First reagent (Reagent E) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 2 kU/L |
| Emulgen B-66 | 2.5 g/L |
| | |

| Second reagent (Reagent b) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Unilube MT-0620B | 22.5 g/L |

### Example 32

A kit for the measurement of RLP-C comprising the following first reagent (Reagent E) and second reagent (Reagent c) was prepared.

| First reagent (Reagent E) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 2 kU/L |
| Emulgen B-66 | 2.5 g/L |
| | |

| Second reagent (Reagent c) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 0.3 kU/L |
| BLAUNON TSP-50 | 100 g/L |

### Example 33

A kit for the measurement of RLP-C comprising the following first reagent (Reagent N) and second reagent (Reagent b) was prepared.

| First reagent (Reagent N) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| Ethylenediamine PO40EO40 | 0.25 g/L |
| | |

| Second reagent (Reagent b) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Unilube MT-0620B | 22.5 g/L |

### Example 34

A kit for the measurement of RLP-C comprising the following first reagent (Reagent Y) and second reagent (Reagent b) was prepared.

| First reagent (Reagent Y) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| COE-301 | 1 kU/L |
| Newcol 1105-SN | 2 g/L |
| | |

| Second reagent (Reagent b) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Unilube MT-0620B | 22.5 g/L |

### Example 35

A kit for the measurement of RLP-C comprising the following first reagent (Reagent AE) and second reagent (Reagent a) was prepared.

| First reagent (Reagent AE) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Persoft EP | 1.5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 36

A kit for the measurement of RLP-C comprising the following first reagent (Reagent AF) and second reagent (Reagent a) was prepared.

| First reagent (Reagent AF) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Persoft EF | 5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 37

A kit for the measurement of RLP-C comprising the following first reagent (Reagent AG) and second reagent (Reagent a) was prepared.

| First reagent (Reagent AG) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Persoft EFT | 5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 38

A kit for the measurement of RLP-C comprising the following first reagent (Reagent AH) and second reagent (Reagent a) was prepared.

| First reagent (Reagent AH) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Persoft EDO | 1.5 g/L |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 39

A kit for the measurement of RLP-C comprising the following first reagent (Reagent AI) and second reagent (Reagent a) was prepared.

| First reagent (Reagent AI) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Newcol 707-SF | 5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 40

A kit for the measurement of RLP-C comprising the following first reagent (Reagent AJ) and second reagent (Reagent a) was prepared.

| First reagent (Reagent AJ) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Newcol 707-SFC | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 41

A kit for the measurement of RLP-C comprising the following first reagent (Reagent AK) and second reagent (Reagent a) was prepared.

| First reagent (Reagent AK) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Newcol 723-SF | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 42

A kit for the measurement of RLP-C comprising the following first reagent (Reagent AL) and second reagent (Reagent a) was prepared.

| First reagent (Reagent AL) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 1 kU/L |
| Epan U108 | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 43

A kit for the measurement of RLP-C comprising the following first reagent (Reagent AM) and second reagent (Reagent a) was prepared.

| First reagent (Reagent AM) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Epan 420 | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 44

A kit for the measurement of RLP-C comprising the following first reagent (Reagent AN) and second reagent (Reagent a) was prepared.

| First reagent (Reagent AN) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Pluronic L-34 | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 45

A kit for the measurement of RLP-C comprising the following first reagent (Reagent AO) and second reagent (Reagent a) was prepared.

| First reagent (Reagent AO) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Pluronic F-68 | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 46

A kit for the measurement of RLP-C comprising the following first reagent (Reagent AP) and second reagent (Reagent a) was prepared.

| First reagent (Reagent AP) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| BLAUNON EP-1461 | 3 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 47

A kit for the measurement of RLP-C comprising the following first reagent (Reagent AQ) and second reagent (Reagent a) was prepared.

| First reagent (Reagent AQ) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Ethylenediamine PO40EO40 | 0.5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 48

A kit for the measurement of RLP-C comprising the following first reagent (Reagent AR) and second reagent (Reagent a) was prepared.

| First reagent (Reagent AR) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Unisafe PKA-5015 | 3 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 49

A kit for the measurement of RLP-C comprising the following first reagent (Reagent AS) and second reagent (Reagent a) was prepared.

| First reagent (Reagent AS) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.01 kU/L |
| Unisafe PKA-5016 | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 50

A kit for the measurement of RLP-C comprising the following first reagent (Reagent AT) and second reagent (Reagent a) was prepared.

| First reagent (Reagent AT) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| EMALEX DAPE-230 | 0.5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 51

A kit for the measurement of RLP-C comprising the following first reagent (Reagent AU) and second reagent (Reagent a) was prepared.

| First reagent (Reagent AU) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.01 kU/L |
| BLAUNON BEO-10 | 0.5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 52

A kit for the measurement of RLP-C comprising the following first reagent (Reagent AV) and second reagent (Reagent a) was prepared.

| First reagent (Reagent AV) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| BLAUNON MXDA-PO4 | 3 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 53

A kit for the measurement of RLP-C comprising the following first reagent (Reagent AW) and second reagent (Reagent a) was prepared.

| First reagent (Reagent AW) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Newcol B4-SN | 0.05 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 54

A kit for the measurement of RLP-C comprising the following first reagent (Reagent AX) and second reagent (Reagent a) was prepared.

| First reagent (Reagent AX) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Newcol B13-SN | 0.05 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 55

A kit for the measurement of RLP-C comprising the following first reagent (Reagent AY) and second reagent (Reagent a) was prepared.

| First reagent (Reagent AY) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Unisafe LM-2 | 1 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 56

A kit for the measurement of RLP-C comprising the following first reagent (Reagent AZ) and second reagent (Reagent a) was prepared.

| First reagent (Reagent AZ) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 1 kU/L |
| Noigen XL-400 | 0.5 g/L |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 57

A kit for the measurement of RLP-C comprising the following first reagent (Reagent BA) and second reagent (Reagent a) was prepared.

| First reagent (Reagent BA) | |
|---|---|
| MOPS (pH 6.5) | 4 g/L |
| EMSE | 0.3 g/L |
| Sodium sulfate Peroxidase | 1 g/L 10 kU/L |
| CHO-CE | 2 kU/L |
| COE-301 | 1kU/L |
| Noigen XL-1000F | 0.5 g/L |
| | |

| Second reagent (Reagent a) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |

### Example 58

A kit for the measurement of RLP-C comprising the following first reagent (Reagent E) and second reagent (Reagent d) was prepared.

| First reagent (Reagent E) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 2 kU/L |
| Emulgen B-66 | 2.5 g/L |
| | |

| Second reagent (Reagent d) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 0.5 kU/L |
| Emulgen A-500 | 100 g/L |

### Example 59

A kit for the measurement of RLP-C comprising the following first reagent (Reagent E) and second reagent (Reagent e) was prepared.

| First reagent (Reagent E) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 2 kU/L |
| Emulgen B-66 | 2.5 g/L |
| | |

| Second reagent (Reagent e) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 0.5 kU/L |
| Newcol 740 | 100 g/L |

### Example 60

A kit for the measurement of RLP-C comprising the following first reagent (Reagent E) and second reagent (Reagent f) was prepared.

| First reagent (Reagent E) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 2 kU/L |
| Emulgen B-66 | 2.5 g/L |
| | |

| Second reagent (Reagent f) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| EMALEX 2425 | 50 g/L |

### Example 61

A kit for the measurement of RLP-C comprising the following first reagent (Reagent E) and second reagent (Reagent g) was prepared.

| First reagent (Reagent E) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 2 kU/L |
| Emulgen B-66 | 2.5 g/L |
| | |

| Second reagent (Reagent g) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Noigen TDS-500F | 75 g/L |

### Example 62

A kit for the measurement of RLP-C comprising the following first reagent (Reagent F) and second reagent (Reagent h) was prepared.

| First reagent (Reagent F) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 1 kU/L |
| Newkalgen GP-120 | 4 g/L |
| | |

| Second reagent (Reagent h) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pluronic F-108 | 75 g/L |

### Example 63

A kit for the measurement of RLP-C comprising the following first reagent (Reagent BB) and second reagent (Reagent i) was prepared.

| First reagent (Reagent BB) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 1 kU/L |
| Noigen XL-400 | 0.75 g/L |
| | |

| Second reagent (Reagent i) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LDL-312 | 1.5 kU/L |
| Emulgen A-500 | 75 g/L |

### Example 64

A kit for the measurement of RLP-C comprising the following first reagent (Reagent BB) and second reagent (Reagent j) was prepared.

| First reagent (Reagent BB) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 1 kU/L |
| Noigen XL-400 | 0.75 g/L |
| | |

| Second reagent (Reagent j) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Newcol 740 | 75 g/L |

### Example 65

A kit for the measurement of RLP-C comprising the following first reagent (Reagent BB) and second reagent (Reagent f) was prepared.

| First reagent (Reagent BB) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 1 kU/L |
| Noigen XL-400 | 0.75 g/L |
| | |

| Second reagent (Reagent f) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| EMALEX 2425 | 50 g/L |

### Example 66

A kit for the measurement of RLP-C comprising the following first reagent (Reagent AN) and second reagent (Reagent k) was prepared.

| First reagent (Reagent AN) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Pluronic L-34 | 1 g/L |
| | |

| Second reagent (Reagent k) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 0.5 kU/L |
| Emulgen A-500 | 50 g/L |

### Example 67

A kit for the measurement of RLP-C comprising the following first reagent (Reagent AN) and second reagent (Reagent l) was prepared.

| First reagent (Reagent AN) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Pluronic L-34 | 1 g/L |
| | |

| Second reagent (Reagent l) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 0.5 kU/L |
| Newcol 740 | 50 g/L |

### Example 68

A kit for the measurement of RLP-C comprising the following first reagent (Reagent AN) and second reagent (Reagent m) was prepared.

| First reagent (Reagent AN) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| Pluronic L-34 | 1 g/L |
| | |

| Second reagent (Reagent m) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Noigen TDS-500F | 50 g/L |

### Example 69

A kit for the measurement of RLP-C comprising the following first reagent (Reagent BC) and second reagent (Reagent n) was prepared.

| First reagent (Reagent BC) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| BIGCHAP | 0.5 g/L |
| | |

| Second reagent (Reagent n) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 2 g/L |

### Example 70

A kit for the measurement of RLP-C comprising the following first reagent (Reagent BD) and second reagent (Reagent n) was prepared.

| First reagent (Reagent BD) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| deoxy-BIGCHAP | 0.5 g/L |
| | |

| Second reagent (Reagent n) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 2 g/L |

### Example 71

A kit for the measurement of RLP-C comprising the following first reagent (Reagent BE) and second reagent (Reagent n) was prepared.

| First reagent (Reagent BE) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| CHAPSO | 0.5 g/L |
| | |

| Second reagent (Reagent n) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 2 g/L |

### Example 72

A kit for the measurement of RLP-C comprising the following first reagent (Reagent BD) and second reagent (Reagent o) was prepared.

| First reagent (Reagent BD) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| deoxy-BIGCHAP | 0.5 g/L |
| | |

| Second reagent (Reagent o) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Unilube MT-0620B | 2 g/L |

### Example 73

A kit for the measurement of RLP-C comprising the following first reagent (Reagent BD) and second reagent (Reagent p) was prepared.

| First reagent (Reagent BD) | |
|---|---|
| MOPS (pH 7.5) | 4 g/L |
| EMSE | 0.3 g/L |
| Sodium sulfate | 1 g/L |
| Peroxidase | 10 kU/L |
| CHO-CE | 2 kU/L |
| Chemically modified LPL | 0.025 kU/L |
| deoxy-BIGCHAP | 0.5 g/L |
| | |

| Second reagent (Reagent p) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| CEN | 20 kU/L |
| Emulgen A-500 | 40 g/L |

### Example 74

A kit for the measurement of RLP-C comprising the following first reagent (Reagent BF) and second reagent (Reagent q) was prepared.

| First reagent (Reagent BF) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| BIGCHAP | 0.5 g/L |
| | |

| Second reagent (Reagent q) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| CEN | 20 kU/L |
| Pronon B-208 | 2 g/L |

### Example 75

A kit for the measurement of RLP-C comprising the following first reagent (Reagent BF) and second reagent (Reagent p) was prepared.

| First reagent (Reagent BF) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| BIGCHAP | 0.5 g/L |
| | |

| Second reagent (Reagent p) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| CEN | 20 kU/L |
| Emulgen A-500 | 40 g/L |

### Example 76

A kit for the measurement of RLP-C comprising the following first reagent (Reagent BF) and second reagent (Reagent r) was prepared.

| First reagent (Reagent BF) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 0.75 g/L |
| Chemically modified LPL | 0.05 kU/L |
| BIGCHAP | 0.5 g/L |
| | |

| Second reagent (Reagent r) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| CEN | 20 kU/L |
| EMALEX 2425 | 2 g/L |

### Example 77

Measurement of the concentration of RLP-C in human serum (45 samples) was carried out using the kit described in Reference Example 1 (hereinafter referred to as the control kit) and the kits of Examples 2 to 4. Reference Example 2 illustrates the data showing the capability of the control kit described in Reference Example 1.

### (1) Preparation of a Calibration Curve

A calibration curve which shows the relationship between the RLP-C and "the absorbance" was prepared with respect to each of the control kit and the kits of Examples 2 to 4 using, as standard solutions, a physiological saline (RLP-C: 0.0 mg/dL) and a standard serum found to have an RLP-C concentration of 35.0 mg/dL by the measurement using RLP-cholesterol "JIMRO" II (JIMRO Co., Ltd.), and Hitachi-7170S autoanalyzer.

"The absorbance" herein refers to the value obtained based on the two absorbances (E1 and E2) measured in the following reaction and by subtracting E1 from E2.

To a reaction cell were added a standard solution (3.2 µL) and the first reagent (150 µL), and the resulting mixture was incubated at 37°C for 5 minutes. The absorbance of the reaction solution (E1) was measured at a main wavelength of 600 nm and a sub-wavelength of 700 nm. Then, the second reagent (50 µL) was added to the reaction solution, followed by further incubation at 37°C for 5 minutes, and the absorbance of the reaction solution (E2) was measured at a main wavelength of 600 nm and a sub-wavelength of 700 nm.

### (2) Measurement of "the Absorbance" of Human Serum Samples

"The absorbance" of the samples was measured in the same manner as in the calculation method of "the absorbance" of (1), except that the human serum samples (45 samples) were used in place of the standard solutions used in the preparation of the calibration curve of (1).

### (3) Determination of the Concentration of RLP-C in Human Serum Samples

The concentration of RLP-C in each of the samples was measured from "the absorbance" measured in (2) and the calibration curve prepared in (1).

The correlation coefficients between the measurements using the control kit and the kits of Examples 2 to 4 were calculated from the concentration of RLP-C in each of the samples obtained by the measurement using the control kit and the concentration of RLP-C in each of the samples obtained by the measurement using the kits of Examples 2 to 4. The results are shown in Table 1.

**Table 1**

| Kit | Correlation coefficient to the control kit |
|---|---|
| Example 2 | 0.8505 |
| Example 3 | 0.8808 |
| Example 4 | 0.8447 |

As shown in Table 1, the correlation between the measurement using the control kit and the measurement using the kits of Examples 2 to 4 was good.

### Example 78

The RLP-C concentration in fresh human serum samples (40 samples) was measured using a kit for the measurement of RLP-C (RLP-cholesterol "JIMRO" II; JIMRO Co., Ltd.) according to the operations described in the manual attached thereto. Separately, the concentration of RLP-C in the above samples (40 samples) was measured in the same manner as in Example 77 using the control kit and the kits of Examples 1 and 5 to 30. The correlation coefficients between RLP-cholesterol "JIMRO" II and the kits of Examples 1, 5 to 11 and 14 to 30 were calculated from the concentration of RLP-C in each of the 40 samples measured using the control kit and the kits of Examples 1, 5 to 11 and 14 to 30 and the concentration of RLP-C in each of the 40 samples measured using RLP-cholesterol "JIMRO" II. The results are shown in Table 2.

**Table 2**

| Kit | Correlation coefficient to JIMRO |
|---|---|
| Control kit | 0.9638 |
| Example 1 | 0.9341 |
| Example 5 | 0.9210 |
| Example 6 | 0.9454 |
| Example 7 | 0.9379 |
| Example 8 | 0.8896 |
| Example 9 | 0.9183 |
| Example 10 | 0.8990 |
| Example 11 | 0.8179 |
| Example 14 | 0.9436 |
| Example 15 | 0.9281 |
| Example 16 | 0.9013 |
| Example 17 | 0.8447 |
| Example 18 | 0.9546 |
| Example 19 | 0.9384 |
| Example 20 | 0.8635 |
| Example 21 | 0.9271 |
| Example 22 | 0.9598 |
| Example 23 | 0.9224 |
| Example 24 | 0.8721 |
| Example 25 | 0.9280 |
| Example 26 | 0.9209 |
| Example 27 | 0.9107 |
| Example 28 | 0.9464 |
| Example 29 | 0.8663 |
| Example 30 | 0.9244 |

As shown in Table 2, the correlation between the measurement using the control kit and the kits of Examples 1, 5 to 11 and 14 to 30 and the measurement using RLP-cholesterol "JIMRO" II was good.

### Example 79

The concentration of RLP-C in human serum samples (40 samples) was measured in the same manner as in Example 77 using the control kit and the kits of Examples 1 and 5 to 76. The correlation coefficients between the control kit and the kits of Examples 1 and 5 to 76 were calculated from the concentration of RLP-C in each of the 40 samples measured using the kits of Examples 1 and 5 to 76 and the concentration of RLP-C in each of the 40 samples measured using the control kit. The results are shown in Table 3.

**Table 3**

| Kit | Correlation coefficient to the control kit | Kit | Correlation coefficient to the control kit | Kit | Correlation coefficient to the control kit |
|---|---|---|---|---|---|
| Ex. 1 | 0.9701 | Ex. 29 | 0.8957 | Ex. 54 | 0.8978 |
| Ex. 5 | 0.9396 | Ex. 30 | 0.9785 | Ex. 55 | 0.9191 |
| Ex. 6 | 0.9716 | Ex. 31 | 0.9705 | Ex. 56 | 0.9612 |
| Ex. 7 | 0.9593 | Ex. 32 | 0.8942 | Ex. 57 | 0.9596 |
| Ex. 8 | 0.8917 | Ex. 33 | 0.9739 | Ex. 58 | 0.9246 |
| Ex. 9 | 0.9215 | Ex. 34 | 0.8905 | Ex. 59 | 0.9280 |
| Ex. 10 | 0.9088 | Ex. 35 | 0.8376 | Ex. 60 | 0.8858 |
| Ex. 11 | 0.8427 | Ex. 36 | 0.9304 | Ex. 61 | 0.8204 |
| Ex. 12 | 0.8071 | Ex. 37 | 0.9179 | Ex. 62 | 0.8103 |
| Ex. 13 | 0.8416 | Ex. 38 | 0.8483 | Ex. 63 | 0.9310 |
| Ex. 14 | 0.9660 | Ex. 39 | 0.8047 | Ex. 64 | 0.9464 |
| Ex. 15 | 0.9619 | Ex. 40 | 0.8233 | Ex. 65 | 0.9014 |
| Ex. 16 | 0.9173 | Ex. 41 | 0.8606 | Ex. 66 | 0.8936 |
| Ex. 17 | 0.8486 | Ex. 42 | 0.9525 | Ex. 67 | 0.8928 |
| Ex. 18 | 0.9657 | Ex. 43 | 0.8797 | Ex. 68 | 0.8061 |
| Ex. 19 | 0.9563 | Ex. 44 | 0.9164 | Ex. 69 | 0.8067 |
| Ex. 20 | 0.8847 | Ex. 45 | 0.9089 | Ex. 70 | 0.8724 |
| Ex. 21 | 0.9571 | Ex. 46 | 0.9295 | Ex. 71 | 0.8974 |
| Ex. 22 | 0.9655 | Ex. 47 | 0.9293 | Ex. 72 | 0.9594 |
| Ex. 23 | 0.9255 | Ex. 48 | 0.9170 | Ex. 73 | 0.9388 |
| Ex. 24 | 0.8891 | Ex. 49 | 0.8402 | Ex. 74 | 0.9369 |
| Ex. 25 | 0.9383 | Ex. 50 | 0.9203 | Ex. 75 | 0.9021 |
| Ex. 26 | 0.9695 | Ex. 51 | 0.8671 | Ex. 76 | 0.8884 |
| Ex. 27 | 0.9455 | Ex. 52 | 0.9440 | | |
| Ex. 28 | 0.9688 | Ex. 53 | 0.8845 | | |

As shown in Table 3, the correlation between the measurement using the control kit and the measurement using the kits of Examples 1 and 5 to 76 was good.

### Example 80

A kit for the measurement of RLP-C comprising the following first reagent (Reagent E) and second reagent (Reagent s) was prepared.

| First reagent (Reagent E) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 2 kU/L |
| Emulgen B-66 | 2.5 g/L |
| | |

| Second reagent (Reagent s) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |
| PLDP | 5 kU/L |

### Example 81

A kit for the measurement of RLP-C comprising the following first reagent (Reagent E) and second reagent (Reagent t) was prepared.

| First reagent (Reagent E) | |
|---|---|
| MOPS (pH 6.5) | 4 g/L |
| EMSE | 0.3 g/L |
| Sodium sulfate | 1 g/L |
| Peroxidase | 10 kU/L |
| CHO-CE | 2 kU/L |
| COE-301 | 2 kU/L |
| Emulgen B-66 | 2.5 g/L |

| Second reagent (Reagent t) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |
| PLA2 | 3 kU/L |

### Example 82

A kit for the measurement of RLP-C comprising the following first reagent (Reagent W) and second reagent (Reagent s) was prepared.

| First reagent (Reagent W) | |
|---|---|
| MOPS (pH 6.5) | 4 g/L |
| EMSE | 0.3 g/L |
| Peroxidase | 10 kU/L |
| CHO-CE | 2 kU/L |
| Dextran sodium sulfate | 1.5 g/L |
| Magnesium nitrate hexahydrate | 1.5 g/L |
| COE-301 | 1 kU/L |
| Newcol 707-SN | 4 g/L |
| | |

| Second reagent (Reagent s) | |
|---|---|
| MOPS (pH 6.8) | 4 g/L |
| 4-Aminoantipyrine | 0.5 g/L |
| Peroxidase | 20 kU/L |
| CHO-CE | 2 kU/L |
| LPL-312 | 1.5 kU/L |
| Pronon B-208 | 20 g/L |
| PLDP | 5 kU/L |

### Example 83

The concentration of RLP-C in each of human serum samples (50 samples) was measured on Hitachi-7170S autoanalyzer in the same manner as in Example 77 using the control kit and the kits of Examples 5, 23 and 80 to 82. The correlation coefficients between the control kit and the kits of Examples 5, 23 and 80 to 82 were calculated from the concentration of RLP-C in each of the 50 samples measured using the control kit and the concentration of RLP-C in each of the 50 samples measured using the kits of Examples 5, 23 and 80 to 82. The results are shown in Table 4.

**Table 4**

| Kit | Phospholipid-hydrolyzing enzyme | Correlation coefficient to the control kit |
|---|---|---|
| Example 5 | - | 0.9054 |
| Example 80 | PLDP | 0.9505 |
| Example 81 | PLA2 | 0.9229 |
| Example 23 | - | 0.8583 |
| Example 82 | PLDP | 0.9219 |

As shown in Table 4, it was revealed, from the comparison of the measurement using the kit of Example 5 and the measurement using the kits of Examples 80 and 81, and also from the comparison of the measurement using the kit of Example 23 and the measurement using the kit of Example 82, that the correlation coefficient to the measurement using the control kit was improved by adding a phospholipid-hydrolyzing enzyme to the second reagent.

### Reference Example 1

A kit for the determination of RLP-C comprising the following first reagent and second reagent was prepared.

| First reagent | |
|---|---|
| MOPS (pH 6.8) | 20 mmol/L |
| TOOS | 0.3 g/L |
| Sodium sulfate | 2 g/L |
| Pronon B-208 | 8 g/L |
| Wondersurf RL-100 | 1 g/L |
| Peroxidase | 10 kU/L |
| Ascorbate oxidase | 2 kU/L |
| | |

| Second reagent | |
|---|---|
| MOPS (pH 6.8) | 20 mmol/L |
| 4-Aminoantipyrine | 0.5 g/L |
| Wondersurf RL-100 | 1 g/L |
| Peroxidase | 10 kU/L |
| Cholesterol esterase | 1 kU/L |
| (Toyobo Co., Ltd.) | |
| Cholesterol oxidase | 3 kU/L |
| (Kyowa Hakko Kogyo Co., Ltd.) | |
| Phospholipase D | 5 kU/L |
| (Asahi Kasei Corporation) | |

### Reference Example 2 Determination of RLP-C

RLP-C in each of fresh human serum samples (64 samples) was measured on Hitachi-7170 autoanalyzer in the following manner using the kit of Reference Example 1.

### (1) Preparation of a Calibration Curve

A standard serum solution found to have an RLP-C concentration of 32.4 mg/dL by the measurement using RLP-cholesterol "JIMRO" II (JIMRO Co., Ltd.) was used as a standard solution, and serum dilutions prepared by appropriately diluting the standard serum solution were used as samples for preparation of a calibration curve.

A calibration curve was prepared by measuring the concentration of RLP-C in each of the serum dilutions on Hitachi-7170 autoanalyzer using the kit of Reference Example 1 as a measurement kit in the following manner.

To a reaction cell were added the serum dilution (3.8 µL) and the first reagent (0.18 mL), and the resulting mixture was incubated at 37°C for 5 minutes. The absorbance of the reaction solution (E1) was measured at a main wavelength of 600 nm and a sub-wavelength of 700 nm (photometric point: 16). Then, the second reagent (0.06 mL) previously heated to 37°C was added to the reaction solution, followed by further incubation at 37°C for 5 minutes, and the absorbance of the reaction solution (E2) was measured at a main wavelength of 600 nm and a sub-wavelength of 700 nm (photometric point: 32). A calibration curve was prepared based on the relationship between the change in absorbance by the reaction (E2-E1) and the RLP-C concentration in the serum dilutions.

### (2) Determination of RLP-C in Fresh Human Serum (64 Samples)

Reaction was carried out in the same manner as in (1) using fresh human serum (64 samples) in place of the standard serum solution, and the concentration of RLP-C in each of the samples was determined from the absorbance of the reaction solution after the reaction and the calibration curve prepared in (1).

### (3) Correlation with the Measurement Using RLP-cholesterol "JIMRO" II (JIMRO Co., Ltd.)

The concentration of RLP-C in each of the above 64 fresh human serum samples was determined using RLP-cholesterol "JIMRO" II (JIMRO Co., Ltd.).

The correlation between the values obtained in the above (2) and (3) with respect to each of the samples was evaluated in terms of correlation coefficient. As the result, the obtained correlation coefficient was 0.9513. The correlation diagram is shown in Fig. 1.

### Industrial Applicability

The present invention provides a method, a reagent and a kit for the measurement of cholesterol in remnant-like particles which are useful for the diagnosis of diseases such as arteriosclerosis.

## Claims

1. A method for the measurement of cholesterol in remnant-like particles (hereinafter abbreviated as RLP) (hereinafter abbreviated as RLP-C) in a sample, which comprises carrying out the following steps (i) to (iii) successively:
(i) eliminating cholesterol in lipoproteins other than RLP by reacting the sample with cholesterol oxidase, or cholesterol dehydrogenase and oxidized coenzyme, in an aqueous medium comprising the sample and a surfactant selected from the group consisting of polyoxyethylene alkyl ether phosphates, polyoxyethylene alkyl ether sulfuric acid ester salts, higher alcohol sulfuric acid ester salts, polyoxyethylene polycyclic phenyl ethers with an HLB value of less than 16.0, polyoxyethylene polycyclic phenyl ether sulfuric acid ester salts, polyoxyethylene-polyoxyalkylene polycyclic phenyl ethers, ethylenediamine-polyoxyethylene polyoxypropylene copolymers, polyoxyethylene alkylphenyl ethers, polyoxyethylene alkylphenyl ether sulfuric acid ester salts, polyoxyethylene short-chain branched alkyl ethers, polyoxyethylene-polyoxypropylene copolymers having a polyoxypropylene molecular weight of less than 2,750, polyoxyethylene-polyoxyalkylene short-chain branched alkyl ethers, polyoxyethylene-polyoxyalkylene alkyl ethers, bile acid derivatives, polyoxyethylene bisphenol-A-ethers, polyoxyethylene aryl ether sulfuric acid ester salts and polyoxypropylene meta-xylene diamines (hereinafter the surfactant is referred to as surfactant A) ;
(ii) reacting the reaction solution from which cholesterol in lipoproteins other than RLP has been eliminated in the above step (i) with cholesterol esterase and cholesterol oxidase, or cholesterol esterase, cholesterol dehydrogenase and oxidized coenzyme, in the presence of a surfactant selected from the group consisting of polyoxyethylene-polyoxybutylene copolymers, polyoxyethylene polycyclic phenyl ethers with an HLB value of not less than 16.0, polyoxyethylene-polyoxyalkylene long-chain branched alkyl ethers, polyoxyethylene-polyoxypropylene copolymers having a polyoxypropylene molecular weight of not less than 2,750 and polyoxyethylene long-chain branched alkyl ethers (hereinafter the surfactant is referred to as surfactant B) to form hydrogen peroxide or reduced coenzyme; and
(iii)measuring the hydrogen peroxide or reduced coenzyme formed in the above step (ii).

2. The method according to Claim 1, wherein step (i) is a step of eliminating cholesterol in lipoproteins other than RLP by reacting the sample with cholesterol dehydrogenase, oxidized coenzyme and reduced coenzyme oxidase in an aqueous medium comprising the sample and surfactant A.

3. The method according to Claim 1 or 2, wherein the reaction of step (i) is carried out in the presence of a polyanion.

4. The method according to Claim 3, wherein surfactant A is a surfactant selected from the group consisting of ethylenediamine-polyoxyethylene polyoxypropylene copolymers, polyoxyethylene alkylphenyl ethers, polyoxyethylene alkylphenyl ether sulfuric acid ester salts, polyoxyethylene short-chain branched alkyl ethers, polyoxyethylene-polyoxypropylene copolymers having a polyoxypropylene molecular weight of less than 2,750, polyoxyethylene-polyoxyalkylene short-chain branched alkyl ethers and bile acid derivatives [hereinafter the surfactant is referred to as surfactant (A1)].

5. The method according to Claim 1 or 2, wherein the reaction of step (i) is carried out in the presence of a polyanion and a divalent metal ion, or in the presence of an antibody selected from the group consisting of anti-apoB antibodies and anti-apoE antibodies.

6. The method according to Claim 5, wherein surfactant A is a surfactant selected from the group consisting of polyoxyethylene alkyl ether sulfuric acid ester salts, polyoxyethylene polycyclic phenyl ether sulfuric acid ester salts, polyoxyethylene-polyoxyalkylene polycyclic phenyl ethers, ethylenediamine-polyoxyethylene polyoxypropylene copolymers, polyoxyethylene alkylphenyl ethers, polyoxyethylene-polyoxypropylene copolymers having a polyoxypropylene molecular weight of less than 2,750, polyoxyethylene-polyoxyalkylene short-chain branched alkyl ethers, polyoxyethylene-polyoxyalkylene alkyl ethers and bile acid derivatives [hereinafter the surfactant is referred to as surfactant (A2)].

7. The method according to any of Claims 1 to 6, wherein step (i) is carried out in the presence of cholesterol esterase.

8. The method according to any of Claims 1 to 7, wherein step (ii) is carried out in the presence of a phospholipid-hydrolyzing enzyme.

9. The method according to any of Claims 1 to 8, wherein hydrogen peroxide is eliminated in the presence of a combination of peroxidase and one of the two oxidative coupling-type chromogens used for oxidative coupling coloring reaction, or in the presence of catalase.

10. A reagent for the measurement of RLP-C in a sample comprising cholesterol esterase, cholesterol oxidase, a reagent for the elimination of hydrogen peroxide, a reagent for the measurement of hydrogen peroxide, surfactant A and surfactant B.

11. A reagent for the measurement of RLP-C in a sample comprising cholesterol esterase, cholesterol dehydrogenase, oxidized coenzyme, surfactant A and surfactant B.

12. A reagent for the measurement of RLP-C in a sample comprising cholesterol esterase, cholesterol dehydrogenase, oxidized coenzyme, reduced coenzyme oxidase, a reagent for the elimination of hydrogen peroxide, a reagent for the measurement of hydrogen peroxide, surfactant A and surfactant B.

13. The reagent according to Claim 10 or 12, wherein the reagent for the elimination of hydrogen peroxide is a reagent comprising peroxidase and one of the two oxidative coupling-type chromogens used for oxidative coupling coloring reaction, or a reagent comprising catalase.

14. The reagent according to any of Claims 10 to 13, further comprising a polyanion.

15. The reagent according to Claim 14, wherein surfactant A is surfactant (A1).

16. The reagent according to any of Claims 10 to 13, further comprising a reagent comprising a polyanion and a divalent metal ion, or a reagent comprising an antibody selected from the group consisting of anti-apoB antibodies and anti-apoE antibodies.

17. The reagent according to Claim 16, wherein surfactant A is surfactant (A2).

18. The reagent according to any of Claims 10 to 17, further comprising a phospholipid-hydrolyzing enzyme.

19. A kit for the measurement of RLP-C in a sample which comprises a first reagent comprising cholesterol oxidase, a reagent for the elimination of hydrogen peroxide and surfactant A and a second reagent comprising a reagent for the measurement of hydrogen peroxide and surfactant B, said kit further comprising cholesterol esterase in either or both of the first reagent and the second reagent.

20. A kit for the measurement of RLP-C in a sample which comprises a first reagent comprising cholesterol dehydrogenase, oxidized coenzyme and surfactant A and a second reagent comprising surfactant B, said kit further comprising cholesterol esterase in either or both of the first reagent and the second reagent.

21. A kit for the measurement of RLP-C in a sample which comprises a first reagent comprising cholesterol dehydrogenase, oxidized coenzyme, reduced coenzyme oxidase, a reagent for the elimination of hydrogen peroxide and surfactant A and a second reagent comprising a reagent for the measurement of hydrogen peroxide and surfactant B, said kit further comprising cholesterol esterase in either or both of the first reagent and the second reagent.

22. The kit according to Claim 19 or 21, wherein the reagent for the elimination of hydrogen peroxide is a reagent comprising peroxidase and one of the two oxidative coupling-type chromogens used for oxidative coupling coloring reaction, or a reagent comprising catalase.

23. The kit according to any of Claims 19 to 22, wherein the first reagent further comprises a polyanion.

24. The kit according to Claim 23, wherein surfactant A is surfactant (A1).

25. The kit according to any of Claims 19 to 22, wherein the first reagent further comprises a polyanion and a divalent metal ion, or an antibody selected from the group consisting of anti-apoB antibodies and anti-apoE antibodies.

26. The kit according to Claim 25, wherein surfactant A is surfactant (A2).

27. The kit according to any of Claims 19 to 26, wherein the second reagent further comprises a phospholipid-hydrolyzing enzyme.
